(19) Europäisches Patentamt
European Patent Office
Office européen des brevets

(11) **EP 2 709 718 B1**

(12) **EUROPÄISCHE PATENTSCHRIFT**

(45) Veröffentlichungstag und Bekanntmachung des Hinweises auf die Patenterteilung:
**17.08.2016   Patentblatt 2016/33**

(21) Anmeldenummer: **13736506.0**

(22) Anmeldetag: **20.06.2013**

(51) Int Cl.:
*A61N 1/32* (2006.01)   *A61N 1/40* (2006.01)

(86) Internationale Anmeldenummer:
**PCT/EP2013/062920**

(87) Internationale Veröffentlichungsnummer:
**WO 2013/190057 (27.12.2013 Gazette 2013/52)**

(54) **THERAPIEVORRICHTUNG ZUR ERZEUGUNG VON ELEKTROMAGNETISCHEN SCHWINGUNGEN**

THERAPY DEVICE FOR PRODUCING ELECTROMAGNETIC VIBRATIONS

APPAREIL DE THÉRAPIE POUR LA PRODUCTION DE VIBRATIONS ÉLECTROMAGNÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.06.2012   DE 202012006092 U**
**15.11.2012   DE 202012010985 U**
**07.03.2013   DE 102013102275**

(43) Veröffentlichungstag der Anmeldung:
**26.03.2014   Patentblatt 2014/13**

(60) Teilanmeldung:
**15166253.3 / 2 944 350**

(73) Patentinhaber: **Baklayan, Alan, E.**
**80331 München (DE)**

(72) Erfinder: **Baklayan, Alan, E.**
**80331 München (DE)**

(74) Vertreter: **Flügel Preissner Schober Seidel Patentanwälte PartG mbB**
**Nymphenburger Strasse 20a**
**80335 München (DE)**

(56) Entgegenhaltungen:
**EP-A1- 0 241 695        EP-A1- 0 293 068**
**DE-U1-202010 010 700    GB-A- 1 577 834**
**US-A- 5 891 185         US-A1- 2003 176 901**
**US-A1- 2005 090 867     US-A1- 2006 190 063**
**US-A1- 2007 179 564     US-B1- 6 424 864**

**EP 2 709 718 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft eine Therapievorrichtung zur Erzeugung von elektromagnetischen Schwingungen und ein Verfahren zur Steuerung einer Therapievorrichtung.

Aus dem Stand der Technik sind Geräte zur Behandlung von Patienten mit energetischen Schwingungen bekannt. Beispielsweise DE 20 2010 010 700 U1 beschreibt ein solches Behandlungsgerät. Das Gerät erzeugt elektrische Schwingungen, die über ein Kabel an eine Elektrode geleitet werden. Der Patient berührt die Elektrode und die von dem Gerät erzeugten elektrischen Schwingungen werden in den Körper des Patienten geleitet. Die elektrischen Schwingungen wechselwirken mit elektrophysiologischen Schwingungen des Körpers. Elektrische Schwingungen treten in jedem Organismus auf. Die von dem Behandlungsgerät erzeugten elektrischen Schwingungen sollen die sich in dem Körper befindenden elektrophysiologischen Schwingungen anregen, verstärken oder unterdrücken, je nachdem, ob die elektrophysiologischen Schwingungen gewünscht oder nicht gewünscht sind. Nicht gewünschte elektrophysiologische Schwingungen können für Krankheiten oder andere Störungen des Organismus verantwortlich sein. Werden elektrophysiologische Schwingungen durch das Behandlungsgerät verstärkt, können positive Effekte der gewünschten elektrophysiologischen Schwingungen verstärkt werden.

US 2005/090867 A1 beschreibt ein Verfahren zur Verringerung der Belastung auf das Herz eines Patienten. Dazu werden Muskeln außerhalb des Herzens zur Bewegung stimuliert, wobei die Stimulation auf den Herzschlag abgestimmt ist. Durch die Kontraktion der stimulierten Muskeln soll eine Pumpwirkung erzielt werden, welche die Pumpleistung des Herzens unterstützt. Dazu wird ein Pulsgenerator verwendet, der mit einer aktiven Elektrode und einer neutralen Elektrode verbunden ist. Der Pulsgenerator erzeugt eine Wechselspannung, die über die aktive Elektrode auf den Patienten übertragen wird. Die neutrale Elektrode bewirkt eine Erdung des Patienten. Dauer, Frequenz, Form und Amplitude der angelegten Spannung kann mit Hilfe von Schaltern geändert werden. Dabei wird die Spannung derart verändert, dass sie mit dem mit einem Elektrokardiogramm gemessenen Herzschlag in einer bestimmten zeitlichen Relation steht.

US 6,424,864 B1 offenbart ein Verfahren und eine Vorrichtung zur Therapie mit Hilfe von niederfrequenten elektrischen Strömen, elektromagnetischen Schwingungen oder akustischen Wellen. Es werden Schwingungen mit Frequenzen von 1 Hz bis 10000 Hz verwendet, wobei bestimmte Frequenzen für bestimmte Krankheiten besondere Wirkungen erzielen sollen. Die Vorrichtung weist einen Oszillator zum Erzeugen eines Stroms mit niedriger Energie und niedriger Frequenz. Der Wechselstrom kann durch eine Frequenzsteuerung zwischen 14 Hz und 1001 Hz variiert werden. Der Oszillator weist eine Oszillationseinrichtung mit einem Hochfrequenzoszillator und einem Frequenzkonvertierer auf. Der Fequenzkonvertierer ist mit einer Therapieelektrode und einer inaktiven Elektrode verbunden, die gegenüberliegend an einem Körperteil des Patienten angebracht sind.

US 2006/190063 A1 beschreibt ein Verfahren und eine Vorrichtung zum Induzieren von Hyperthermie in einen Zielbereich. Dazu werden in dem Zielbereich Partikel oder andere Substanzen hinzugefügt, welche die von der Vorrichtung ausgesandte Strahlung absorbieren und sich so erhitzen. Das Verfahren und die Vorrichtung können in vitro und in vivo angewandt werden. Ein Transmitter steht mit einem Transmissionskopf in elektrischer Verbindung. Die von einem ersten Signalgenerator erzeugte Frequenz und die von einem zweiten Signalgenerator erzeugte Frequenz werden in einem Kombinierer je nach Einstellung kombiniert und an den Transmissionskopf ausgegeben. Der Transmissionskopf ist gegenüber einem Empfangskopf angeordnet. Der Empfangskopf ist mit einem Empfänger verbunden, der zwei einstellbare Schwingkreise aufweist, die jeweils über einen Frequenzsplitter mit dem Empfangskopf verbunden sind.

GB 1 577 834 A beschreibt eine Vorrichtung zum Erzeugen von Wechselströmen für die elektrotherapeutische Behandlung. Ein Frequenzgenerator erzeugt Pulse mit einer Frequenz von 5 kHz. Ein weiterer Oszillator kann einstellbare Frequenzen in einem Bereich von 1 kHz bis 10 kHz erzeugen. Über Tiefpassfilter sowie Verstärker sind die Oszillatoren mit Elektroden verbunden. Über einen Phasendetektor und einen Tiefpassfilter sind die Oszillatoren miteinander verbunden, wobei mit Hilfe eines Niedrigfrequenzoszillators die Phase zwischen den beiden Oszillatoren verändert werden kann.

US 2003/176901 A1 offenbart ein elektrotherapeutisches Gerät zur Behandlung eines Körpers mit elektrischen Strömen, die innerhalb eines Frequenzbereiches von 1 kHz bis 100 kHz simultan amplituden- und frequenzmoduliert werden. Die Frequenzen werden dabei so verändert, dass sie ein Frequenzband von 1 bis 3 Oktaven bilden. Innerhalb des Frequenzbandes werden die Frequenzen kontinuierlich oder schrittweise verändert, wobei mit sich ändernder Frequenz auch die Amplitude variiert wird. Werden zwei unabhängige Ströme an den Körper angelegt, so unterscheiden sich die Frequenzen der beiden Ströme zwischen 1/60 Hz und 1/5 Hz. Das elektrotherapeutische Gerät umfasst mehrere Einheiten, die jeweils einen Stromgenerator aufweisen, wobei die Amplitude des Stromes über ein Stellglied einstellbar ist. Darüber hinaus ist ein Oszillator, der mit einem Frequenzsteller verbunden ist, für jede Einheit vorgesehen. Das Stellglied und der Frequenzsteller werden über Ausgänge eines Mikroprozessors, der mit einem Arbeitsspeicher in Datenverbindung steht, angesteuert. Eine Superpositionseinrichtung steuert die einzelnen Einheiten.

US 5,891,185 A beschreibt ein Verfahren und eine Vorrichtung zum Behandeln von Krankheiten des Schluckapparats mit Hilfe elektrischer Stimulation. Die Stimulationsvorrichtung weist einen Mikroprozessor auf, der vier Pulsgeneratoren steuert. Die Pulsgeneratoren sind mit einem Schaltnetzwerk verbunden, welches die Pulsgeneratoren mit einer Vielzahl von Elektroden abhängig von Steuersignalen des Mikroprozessors verbindet. Die Elektroden sind über Drähte mit dem Schaltnetzwerk verbunden. Der Mikroprozessor steuert die Frequenz und die Amplitude der Pulsgeneratoren sowie die Verbindungsdauer der einzelnen Pulsgeneratoren mit den jeweiligen Elektroden. Dadurch ist es möglich, die verschiedenen Elektroden mit verschiedenen Pulsgeneratoren zu verbinden. Die Elektroden werden mit Schleimhäuten des Mund- und Rachenraums verbunden, um Muskeln zur Erzeugung von Schluckbewegungen anzuregen.

EP 0 293 068 A1 beschreibt eine elektrische Therapievorrichtung, welche das Ausscheiden von Auswurf aus der Lunge anregen soll. Die Therapievorrichtung weist zwei Stromerzeugungsmittel auf, die Wechselstrom verschiedener Frequenz und Amplitude erzeugen. Die Stromerzeugungsmittel sind über einen Transformer mit Leitungen verbunden. Der Transformer koppelt induktiv die Stromerzeugungsmittel mit den Leitungen. Die Leitungen sind mit Elektroden verbunden. In jeder der Leitungen sind Kondensatoren vorgesehen, mittels welchen bioelektrische Potentiale, wie beispielsweise ein Elektrokardiogramm, mit Hilfe eines Potentialmessmittels bestimmt werden können. Die Therapievorrichtung kann somit einerseits elektrische Impulse in den Körper eines Patienten einleiten und gleichzeitig elektrische Erregungspotentiale messen. Die Therapievorrichtung wirkt derart, dass an den Elektroden eine Frequenz von beispielsweise 4000 Hz und an den Elektroden eine Frequenz von 4100 Hz anliegt. Aufgrund der Höhe der Frequenz ist die Impedanz der Haut gering, so dass die elektrischen Impulse in den Körper eines Patienten eindringen können, wo sie dann interferieren.

DE 20 2010 010 700 U1 offenbart eine Therapievorrichtung mit drei Frequenzgeneratoren, die an einem Patienten anliegen. In der Nähe des Patienten ist eine geerdete Antenne vorgesehen. Der Erfindung liegt die Aufgabe zugrunde, eine Therapievorrichtung bereitzustellen, die eine besonders gute Behandlung eines Lebewesens ermöglicht.

Die Aufgabe wird durch die Therapievorrichtung nach Anspruch 1 gelöst. Vorteilhafte Ausgestaltungen sind Gegenstand der Ansprüche 2 bis 12. Erfindungsgemäß umfasst die Therapievorrichtung zur Erzeugung von elektromagnetischen Schwingungen wenigstens eine Frequenzgeneratoreinheit. Die Frequenzgeneratoreinheit umfasst einen Frequenzgenerator zur Erzeugung von Wechselspannung, einen ersten Elektrodenausgang zum Anschluss einer ersten Elektrode und einen zweiten Elektrodenausgang zum Anschluss einer zweiten Elektrode. Der Frequenzgenerator weist einen Spannungsausgang auf, an dem die von dem Frequenzgenerator erzeugte Wechselspannung anliegt. Der Frequenzgenerator weist ferner einen Neutralausgang auf, der elektrisch neutral ist. Der erste Elektrodenausgang ist mit dem Spannungsausgang verbunden, während der zweite Elektrodenausgang mit dem Neutralausgang verbunden ist.

Die von der Therapievorrichtung erzeugten elektromagnetischen Schwingungen dienen vorzugsweise der Behandlung von Menschen oder Tieren mit den von dem Frequenzgenerator erzeugten elektrischen Schwingungen. Wie bereits oben dargestellt, lassen sich mit den elektromagnetischen Schwingungen gewünschte elektrophysiologische Effekte im Körper des Patienten verstärken oder abschwächen. Dazu berührt der Patient vorzugsweise eine erste Elektrode, die über ein Kabel mit dem ersten Elektrodenausgang verbunden ist. Der Patient kann die erste Elektrode halten oder die erste Elektrode kann an dem Patienten mittels eines Fixierbands oder Ähnlichem angebracht sein.

Vorzugsweise berührt der Patient auch eine zweite Elektrode, die über ein Kabel mit dem zweiten Elektrodenausgang verbunden ist. Die zweite Elektrode kann wie die erste Elektrode ausgestaltet sein. Die erste und/oder die zweite Elektrode können auch als eine Antenne zur Aussendung von elektromagnetischen Wellen ausgebildet sein.

Der Frequenzgenerator erzeugt eine Wechselspannung, die an dem Spannungsausgang anliegt. Über den ersten Elektrodenausgang wird die Wechselspannung an die erste Elektrode übertragen. Die Spannung kann vorzugsweise zwischen 0,1 V und 20 V, vorzugsweise zwischen 0,1 V und 16 V variiert werden. Der Neutralausgang des Frequenzgenerators ist vorzugsweise geerdet, kann aber auch mit einer konstanten Spannung beaufschlagt sein. Insbesondere kann der Neutralausgang mit einer Wechselspannung beaufschlagt werden, die gegenüber der am Spannungsausgang anliegenden Wechselspannung invertiert wird. Auf diese Weise kann die am Patienten anliegende Wechselspannung in ihrer Amplitude verdoppelt werden. Bevorzugt kann auch der Spannungsausgang mit einer Offsetspannung belegt sein, so dass die Wechselspannung beispielsweise nur mit einer positiven oder negativen Spannung oszilliert. Der Frequenzgenerator hat vorzugsweise einen Innenwiderstand von 100 $\Omega$ bis 100 k$\Omega$, insbesondere von 1 k$\Omega$.

Wenn der Patient sowohl die erste als auch die zweite Elektrode berührt, kann Strom zwischen den beiden Elektroden fließen. Es hat sich gezeigt, dass das Fließen von Strom für die elektrophysiologische Behandlung besonders wirkungsvoll ist. Durch entsprechendes Positionieren der Elektroden lässt sich der von dem Strom durchflossene Teil des Körpers des Patienten bestimmen, um die Wirkung der Behandlung in dem Körperteil je nach Bedarf zu erhöhen und zu vermindern.

Bevorzugt weist die Therapievorrichtung wenigstens einen Behälter zur Aufnahme einer Probe auf, der zwischen dem Spannungsausgang und dem ersten Elektrodenausgang geschaltet ist. Der Behälter ist vorzugsweise aus einem elektrisch leitenden Material, insbesondere Metall, hergestellt.

Der Behälter dient zur Aufnahme von Proben, die die von dem Frequenzgenerator erzeugte Wechselspannung auf eine für die Behandlung des Patienten positive Weise beeinflussen soll. Dies geschieht beispielsweise durch eine Veränderung der Kapazität des Behälters. Die geänderte Kapazität verändert dann die anliegende Wechselspannung. Die Proben können Eigenproben des Patienten, wie beispielsweise Blut oder Speichel, sein oder belastende Substanzen, wie beispielsweise Allergene, Schwermetalle, Viren oder Bakterien, enthalten.

Es ist bevorzugt, dass der erste Elektrodenausgang wenigstens zwei Anschlüsse, insbesondere drei Anschlüsse umfasst, wobei vorzugsweise der zweite Elektrodenausgang einen Anschluss umfasst, der mit einem der Anschlüsse des ersten Elektrodenausgangs eine koaxiale Steckverbindung, insbesondere einen BNC-Anschluss, bildet.

Es ist somit bevorzugt, dass die Therapievorrichtung zwei Anschlüsse, insbesondere Steckanschlüsse, aufweist, an denen die von dem Frequenzgenerator am Elektrodenausgang erzeugte Wechselspannung abgegriffen werden kann. Ferner hat die Therapievorrichtung vorzugsweise eine koaxiale Steckverbindung, bei der insbesondere der Mittelleiter mit dem Spannungsausgang des Frequenzgenerators verbunden ist und weiter vorzugsweise der Außenleiter mit dem Neutralausgang des Frequenzgenerators. An der koaxialen Steckverbindung können daher zwei Elektroden angeschlossen werden. Es ist auch möglich, dass an der koaxialen Steckverbindung lediglich mit dem Neutralausgang des Frequenzgenerators verbunden wird und die Spannung mittels der beiden anderen Anschlüsse des ersten Ausgangs verbunden ist. An der Therapievorrichtung ist vorzugsweise der Stecker der koaxialen Steckverbindung vorgesehen, wohingegen die Elektroden mit der Buchse der Steckverbindung verbunden sind. Eine umgekehrte Anordnung ist auch möglich. Die koaxiale Steckverbindung kann ein BNC-Anschluss, ein SMA-Anschluss oder eine andere Steckverbindung sein, die insbesondere für Hochfrequenzanschlüsse geeignet sind.

Es ist bevorzugt, dass die Elemente der Frequenzgeneratoreinheit innerhalb eines Gehäuses eingebaut sind, wobei die Anschlüsse von außen zugänglich sind. Insbesondere ist auch der Behälter im Gehäuse angebracht, so dass die Proben in den Behälter eingebracht werden können, ohne das Gehäuse zu öffnen. Der Behälter kann vorzugsweise mittels eines Deckels verschlossen werden.

Bevorzugt weist die Therapievorrichtung einen Energiespeicher, insbesondere einen Akkumulator auf, der den Frequenzgenerator mit elektrischer Energie versorgt.

Es ist somit bevorzugt möglich, dass die Therapievorrichtung von einem Stromnetz getrennt betrieben werden kann. Zum einen ermöglicht dies, dass die durch das Stromnetz erzeugte elektromagnetische Schwingung, wie zum Beispiel die 50Hz-Strahlung, vermieden werden kann, und zum anderen benötigt die Therapievorrichtung vorzugsweise keine Sicherungsmaßnahmen, die bei medizinischen Geräten, die an das Stromnetz angeschlossen werden, notwendig sind. Insbesondere die Vermeidung von elektromagnetischen Schwingungen in der Nähe des Patienten hat den positiven Effekt, dass keine externen Schwingungen die Behandlung des Patienten beeinflussen.

Der Energiespeicher kann insbesondere eine wiederaufladbare Batterie sein, die mittels eines Ladegeräts, das an die Therapievorrichtung anschließbar ist, aufgeladen werden kann. Ferner hat das Vorsehen eines Energiespeichers den Vorteil, dass die Therapievorrichtung variabler einsetzbar ist, da sie nicht an das Vorhandensein eines elektrischen Stromnetzes geknüpft ist. Der Energiespeicher ist vorzugsweise auch innerhalb des Gehäuses der Therapievorrichtung angeordnet.

Es ist bevorzugt, dass der Frequenzgenerator zur Erzeugung von Wechselspannung mit unterschiedlichen Frequenzen, insbesondere zwischen 0,1 Hz und 100 MHz, ausgebildet ist, wobei vorzugsweise der Frequenzgenerator geeignet ist, die Spannung und die Art der ausgegebenen Wechselspannung zu verändern.

Die Abdeckung eines großen Frequenzbereichs zwischen 0,1 Hz und 100 MHz, insbesondere zwischen 1 Hz und 10 MHz, hat den Vorteil, dass die Therapievorrichtung in verschiedensten Anwendungsbereichen verwendet werden kann. Die Spannung, die Spitzen-Spitzen-Spannung, die die Amplitudenspannung oder die Effektivspannung, kann vorzugsweise zwischen 0,1 V und 16 V variiert werden. Vorzugsweise kann der Frequenzgenerator eine Wechselspannung mit sinuswellenförmigem, rechteckigem oder dreieckigem Wellenprofil erzeugen. Die einzelnen Wellenprofile haben erfahrungsgemäß jeweils besondere Vorteile bei den jeweiligen Behandlungen. Ferner kann eine monofrequente, sinusförmige Wechselspannung durch Addition einer oder mehrerer Oberschwingungen (bis zur Entstehung einer Rechtecksspannung) mit Hilfe des Frequenzgenerators verändert werden.

Es ist bevorzugt, dass die Therapievorrichtung eine Steuereinrichtung aufweist, welche mit dem Frequenzgenerator verbunden ist, wobei die Frequenz, die Spannung und/oder die Art der ausgegebenen Wechselspannung mittels der Steuereinrichtung steuerbar sind.

Vorzugsweise ist die Steuereinrichtung innerhalb des Gehäuses der Therapievorrichtung angeordnet und insbesondere durch einen Mikroprozessor realisiert. Weiter vorzugsweise ist der Energiespeicher mit der Steuereinrichtung verbunden, so dass der Frequenzgenerator über die Steuereinrichtung mit Energie versorgt wird. Die Steuereinrichtung gibt vorzugsweise die Frequenz, die Spannung und/oder die Art der ausgegebenen Wechselspannung an den Frequenzgenerator aus, der wiederum eine Wechselspannung mit den von der Steuereinrichtung angegebenen Parametern ausgibt.

Es ist bevorzugt, dass die Therapievorrichtung eine Anzeigeeinrichtung und/oder eine Eingabeeinrichtung aufweist. Die Anzeigeeinrichtung zeigt die Frequenz, die Spannung und/oder die Art der ausgegebenen Wechselspannung an, wobei vorzugsweise die Frequenz, die Spannung und/oder die Art der ausgegebenen Wechselspannung mittels der Eingabeeinrichtung einstellbar sind.

Die Eingabeeinrichtung ist vorzugsweise mit der Steuereinrichtung verbunden. Ferner kann die Steuereinrichtung mit der Anzeigeeinrichtung, die beispielsweise ein Display sein kann, verbunden sein und diese ansteuern. Die Anzeigeeinrichtung und die Eingabeeinrichtung sind vorzugsweise an dem Gehäuse der Therapievorrichtung vorgesehen. Die Art der Wechselspannung kann beispielsweise durch einen Begriff oder durch eine visuelle Darstellung der Wechselspannung angezeigt werden. Die Frequenz und/oder die Spannung kann mittels einer numerischen Eingabe eingegeben werden. Es ist aber auch möglich, dass die Steuereinrichtung einen Speicher aufweist, in dem verschiedenste Frequenzen eingespeichert sind, die jeweils mit einer Nummer versehen sind. Durch Eingabe einer Nummer an der Eingabeeinrichtung kann die gewünschte Frequenz eingegeben werden.

Alternativ oder weiter bevorzugt ist der Frequenzgenerator zur Erzeugung von wenigstens zwölf festgelegten Frequenzen ausgebildet. Die Frequenzen liegen in einem Frequenzbereich, der eine untere Grenzfrequenz und eine obere Grenzfrequenz aufweist. Die obere Grenzfrequenz ist doppelt so groß wie die untere Grenzfrequenz, wobei vorzugsweise die obere Grenzfrequenz nicht Teil des Frequenzbereichs ist.

Der Frequenzgenerator ist ausgebildet, zumindest zwölf festgelegte, unterschiedliche erste Frequenzen zu erzeugen. Diese zwölf Frequenzen liegen alle in innerhalb eines ersten Bereichs, dessen Grenzen bevorzugt einem Oktavengesetz folgen. So ist die untere Grenzfrequenz gleich der niedrigsten Frequenz der zwölf ersten Frequenzen. Die obere Grenzfrequenz ist durch die doppelte Frequenz der unteren Grenzfrequenz gebildet, so dass sich ein oktavenspezifisches Verhältnis von 2:1 ergibt. Erfindungsgemäß ist die obere Grenzfrequenz aus dem ersten Bereich ausgeschlossen.

Das Verhältnis der Frequenzen zur unteren Grenzfrequenz 1, 14:13, 9:8, 6:5, 5:4, 4:3, 7:5, 3:2, 8:5, 5:3, 9:5 oder 13:7 beträgt. Ein Verhältnis der Frequenz zur unteren Grenzfrequenz von 2:1 entspricht der ersten Oberschwingung der unteren Grenzfrequenz und kann auch als Frequenz ausgewählt werden. Diese Frequenzverhältnisse entsprechen der Harmonikalischen Lehre und sind besonders geeignet, die Meridiane des Körpers anzuregen. Alternativ kann ein weiteres Frequenzverhältnis gewählt werden. Bei diesem unterscheiden sich benachbarte Frequenzen der zwölf Frequenzen, also die erste zu der zweiten, die zweite zu der dritten und in diesem Sinne weiter, um ein konstantes Verhältnis $\sqrt[12]{2}$. Auch hier ergibt sich ein Verhältnis von Grundfrequenz zu erster Oberschwingung von 2:1.

Gemäß einem weiteren Aspekt der Erfindung umfasst eine Therapievorrichtung zur Erzeugung von elektromagnetischen Schwingungen wenigstens eine Frequenzgeneratoreinheit. Die Frequenzgeneratoreinheit umfasst einen Frequenzgenerator zur Erzeugung von Wechselspannung, einen ersten Elektrodenausgang zum Anschluss einer ersten Elektrode und einen zweiten Elektrodenausgang zum Anschluss einer zweiten Elektrode sowie eine Steuereinrichtung. Die Steuereinrichtung ist eingerichtet, den Frequenzgenerator für einen ersten Zeitraum zu aktivieren und für einen zweiten Zeitraum zu deaktivieren, wobei vorzugweise sich der erste Zeitraum und der zweite Zeitraum periodisch abwechseln. Die Therapievorrichtung weist vorzugsweise die oben genannten Merkmale und/oder Vorteile auf.

Durch das Aktivieren und Deaktivieren des Frequenzgenerators kann der Patient in dem ersten Zeitraum behandelt werden, während in dem zweiten Zeitraum der Patient zur Ruhe kommen kann. Besonders vorteilhaft ist, dass der Patient während des zweiten Zeitraums entladen werden kann, wenn er eine an mit dem Neutralausgang verbundene Handelektrode berührt. Die während des ersten Zeitraums aufgebrachte Ladung und durch Umwelteinflüsse aufgebrachte Ladung können in diesem Fall im zweiten Zeitraum vollständig abfließen. Vorzugsweise ist die Dauer des ersten und/oder des zweiten Zeitraums einstellbar.

Eine derartige Therapievorrichtung stellt die Grundlage für eine effektive Behandlung eines Patienten dar. Beispielsweise können die zwölf Frequenzen den zwölf Hauptmeridianen der Traditionellen Chinesischen Medizin zugeordnet werden, wodurch es dem behandelnden Therapeuten vereinfacht wird, diese Meridiane mit entsprechenden Frequenzen zu aktivieren.

Bei einer bevorzugten Ausführungsform der Erfindung ist die Frequenzgeneratoreinheit ausgebildet, die ersten Frequenzen in konstanten Verhältnissen zueinander zu erzeugen. Die Verhältnisse müssen dabei nicht zwangsläufig identisch sein, sind jedoch bevorzugt konstant, so dass bei Variation einer der zwölf ersten Frequenzen sämtliche weiteren Frequenzen der ersten Frequenzen gemäß den bevorzugten Verhältnissen ebenso geändert werden.

Weiterhin sind die Verhältnisse der ersten Frequenzen untereinander derart ausgebildet, dass sie gleich zu den Verhältnissen sind, die die zwölf Halbtöne einer Oktave der Klanglehre aufweisen. Durch diese Gesetzmäßigkeiten ist es möglich, eine "Tonleiter" der ersten Frequenzen aufzustellen, die der Tonleiter einer Oktave mit 12 Halbtönen gleicht.

Weiterhin ist bevorzugt vorgesehen, dass die ersten Frequenzen zueinander ein Verhältnis aufweisen, das identisch mit einem Halbton und/oder einem Viertelton aus der Klanglehre ist. Die Therapievorrichtung kann daher diskrete Frequenzwerte abgeben, so dass dem Therapeuten die Auswahl der für den aktuellen Patienten passenden Frequenz erleichtert wird.

Bei einer weiteren vorteilhaften Ausführungsform der Erfindung ist die Frequenzgeneratoreinheit ausgebildet, neben den zwölf ersten Frequenzen weitere Frequenzen zu erzeugen. Bevorzugt folgen die weiteren Frequenzen denselben Gesetzmäßigkeiten wie die ersten Frequenzen. Somit liegen die weiteren Frequenzen innerhalb eines weiteren Bereichs, der durch die niedrigste der weiteren Frequenzen nach unten begrenzt wird. Weiterhin ist auch für jeden weiteren Bereich die Obergrenze als doppelte Frequenz der Frequenz der Untergrenze definiert. Auf diese Weise sind die Voraussetzungen vorhanden, die weiteren Frequenzen analog zu aus der Klanglehre bekannten Oktaven um den ersten Bereich herum anzuordnen. Jede weitere Frequenz steht somit in einem festen Verhältnis zu einer der ersten Frequenzen, wobei die jeweils größere Frequenz um ein natürliches Vielfaches von 2 größer als die jeweils kleinere Frequenz ist. Dadurch ergibt sich ein aus der Klanglehre bekanntes Bild, in dem der gesamte Frequenzraum in eine Aneinanderreihung von oktavenähnlichen Bereichen aufgeteilt ist.

Eine derartige Oktavierung der möglichen Frequenzen, mit denen ein Patient behandelt werden kann, macht es für den Therapeuten einfacher, eine zu verwendende Frequenz für die Behandlung zu finden. So kann beispielsweise eine Frequenz, die einem bestimmten Meridian zugeordnet ist, als Ton angesehen werden, der jedoch auch in anderen Oktaven vorkommt. Dies bedeutet, dass beispielsweise eine doppelt oder halb so große Frequenz auf denselben Meridian wirkt. Dem Therapeuten steht somit offen, eine bestmöglichste Frequenz gemäß dem Oktavengesetz auszuwählen.

Bevorzugt ist die obere Grenzfrequenz und/oder die untere Grenzfrequenz des ersten Bereichs bevorzugt manuell anpassbar. Alternativ oder zusätzlich ist die obere Grenzfrequenz und/oder die untere Grenzfrequenz des ersten Bereichs bevorzugt mittels einer automatischen Steuereinrichtung einstellbar.

Die Anpassbarkeit der Grenzen erlaubt eine vorteilhafte Kalibrierung der Therapievorrichtung auf einen bestimmten Patienten. Da die ersten Frequenzen bei allen Patienten meist nicht exakt gleich sind, kann hier der Therapeut korrigierend eingreifen und die ersten Frequenzen und damit den ersten Bereich entsprechend anpassen. Da die Verhältnisse der Frequenzen untereinander bevorzugt konstant sind, verschiebt sich somit lediglich die Frequenzlage als Ganzes, während die grundsätzliche Oktavierung erhalten bleibt.

Es ist bevorzugt, dass die Therapievorrichtung eine zweite Frequenzgeneratoreinheit und insbesondere eine dritte Frequenzgeneratoreinheit aufweist, wobei vorzugsweise die zweite Frequenzgeneratoreinheit und die dritte Frequenzgeneratoreinheit gegeneinander und/oder gegenüber der ersten Frequenzgeneratoreinheit galvanisch getrennt sind.

Die zweite und/oder die dritte Frequenzgeneratoreinheit sind vorzugsweise innerhalb des Gehäuses der Therapievorrichtung angeordnet. Die zweite und/oder die dritte Frequenzgeneratoreinheit sind vorzugsweise wie die oben beschriebene Frequenzgeneratoreinheit ausgebildet und weisen demnach insbesondere jeweils einen Frequenzgenerator, einen ersten Elektrodenausgang und einen zweiten Elektrodenausgang auf, wobei der Frequenzgenerator jeweils einen Spannungsausgang und einen Neutralausgang aufweist. Ferner sind vorzugsweise jeder Frequenzgeneratoreinheit die oben genannten Anschlüsse zugeordnet. Die einzelnen Frequenzgeneratoreinheiten sind voneinander galvanisch (elektrisch) getrennt, so dass sich die Frequenzgeneratoren der jeweiligen Frequenzgeneratoreinheiten nicht gegenseitig beeinflussen können. Eine gegenseitige elektrische Störung, die ungewünschte Interferenzen zur Folge haben kann, kann somit vermieden werden. Vorzugsweise sind die einzelnen Frequenzgeneratoreinheiten zusätzlich gegen elektromagnetische Strahlung abgeschirmt.

Die zweite und/oder dritte Frequenzgeneratoreinheit wird bevorzugt durch die Steuereinrichtung gesteuert. Auch die Energieversorgung sowie die Eingabe und Anzeige der von den Frequenzgeneratoren der zweiten und dritten Frequenzgeneratoreinheit folgen ähnlichen Mechanismen, wie sie im Zusammenhang mit der ersten Frequenzgeneratoreinheit beschrieben worden sind.

Es ist ferner bevorzugt, dass die Steuereinrichtung geeignet ist, die Frequenz, die Spannung und/oder die Art der ausgegebenen Wechselspannung der Frequenzgeneratoren der zweiten Frequenzgeneratoreinheit und/oder der dritten Frequenzgeneratoreinheit zu steuern.

Vorzugsweise hat die von der zweiten Frequenzgeneratoreinheit und/oder dritten Frequenzgeneratoreinheit erzeugte Wechselspannung eine Frequenz, die das $2^Z$-fache der Frequenz der von der ersten Frequenzgeneratoreinheit erzeugten Wechselspannung ist. z ist dabei eine ganze (positive oder negative) Zahl. Das heißt, dass die von der zweiten Frequenzgeneratoreinheit erzeugte Frequenz das Doppelte, Vierfache usw. oder das Einhalbfache, Einviertelfache usw. der Frequenz der von der ersten Frequenzgeneratoreinheit erzeugten Wechselspannung ist. Die Frequenz der von der dritten Frequenzgeneratoreinheit erzeugten Wechselspannung unterscheidet sich von der Frequenz der von der ersten Frequenzgeneratoreinheit erzeugten Wechselspannung auf gleiche Weise. Insbesondere kann die Steuereinrichtung so eingestellt werden, dass bei Änderung der Frequenz der von der ersten Frequenzgeneratoreinheit erzeugten Wechselspannung auch die Frequenzen der von der zweiten oder dritten Frequenzgeneratoreinheit erzeugten Wechselspannung geändert werden. Vorzugsweise ist die Art der Wechselspannung dabei bei allen Frequenzgeneratoreinheiten gleich.

Es ist bevorzugt, dass die Phase der von dem Frequenzgenerator der zweiten Frequenzgeneratoreinheit und/oder dem Frequenzgenerator der dritten Frequenzgeneratoreinheit ausgegebenen Wechselspannung zur Phase der von dem Frequenzgenerator der ersten Frequenzgeneratoreinheit ausgegebenen Wechselspannung verschoben ist, insbesondere um 60°, 90°, 120° oder 180°.

Wenn ein Patient zwei und/oder drei Elektroden, die jeweils mit den verschiedenen Frequenzgeneratoreinheiten verbunden sind, berührt, hat sich gezeigt, dass die Invertierung der Wechselspannungen der einzelnen Frequenzgeneratoreinheiten einen besonders starken therapeutischen Effekt auf den Patienten hat. Beispielsweise gibt die Frequenzgeneratoreinheit eine Wechselspannung mit einer bestimmten Frequenz aus, die zu der von der zweiten Frequenzgeneratoreinheit erzeugten Wechselspannung mit der gleichen Frequenz um 180° phasenverschoben ist. Die von der dritten Frequenzgeneratoreinheit erzeugte Wechselspannung kann gegenüber der von der ersten oder der zweiten Frequenzgeneratoreinheit erzeugten Wechselspannung um 60°, 90°, 120° oder 180° phasenverschoben sein.

Es ist bevorzugt, dass sich die Spannung der von dem Frequenzgenerator der zweiten Frequenzgeneratoreinheit ausgegebenen Wechselspannung und/oder der von dem Frequenzgenerator der dritten Frequenzgeneratoreinheit ausgegebenen Wechselspannung von der Spannung der von dem Frequenzgenerator der ersten Frequenzgeneratoreinheit ausgegebenen Wechselspannung unterscheidet.

Vorzugsweise haben die Spannungen der drei ausgegebenen Frequenzen ein Verhältnis von 4 : 2 : 1. Es sind aber auch andere Frequenzverhältnisse zwischen den verschiedenen Wechselspannungen möglich. Insbesondere können die Spannungen der von den drei Frequenzgeneratoreinheiten erzeugten Wechselspannungen auch gleich sein. Vorzugsweise lassen sich die Spannungen und damit auch die Verhältnisse der Spannungen der von den drei Frequenzgeneratoreinheiten erzeugten Wechselspannungen einstellen, um einen optimalen Behandlungserfolg erzielen zu können.

Es ist bevorzugt, dass die Steuereinrichtung einen Regler umfasst, welcher die Spannung der Wechselspannung derart regelt, dass der Stromfluss zwischen dem ersten Elektrodenausgang und dem zweiten Elektrodenausgang zumindest im Mittel konstant ist, wobei vorzugsweise die Stromstärke einstellbar ist.

Es hat sich gezeigt, dass die Höhe des am oder im Patienten fließenden Stroms wichtig für den Behandlungserfolg ist. Allerdings unterscheidet sich die Leitfähigkeit der Elektrode zu der Haut des Patienten von Patient zu Patient und von Situation zu Situation, da die Beschaffenheit der Haut bei jedem Patienten unterschiedlich ist und die Leitfähigkeit maßgeblich von der Feuchtigkeit zwischen der Haut und der Elektrode abhängt. Um diese Faktoren nicht berücksichtigen zu müssen, kann vorzugsweise die Stromstärke, insbesondere mit Hilfe der Eingabeeinrichtung, an der Therapievorrichtung eingestellt werden. Der Regler übernimmt dann das Festlegen der Spannung. Bei einer guten Leitfähigkeit zwischen der Elektrode und der Haut des Patienten, das heißt bei einem geringern Kontaktwiderstand, wird die am Frequenzgenerator am Spannungsausgang anliegende Spannung geringer sein, als wenn der Kontaktwiderstand zwischen Elektrode und Haut groß ist.

Ferner stellt die Erfindung ein Verfahren zur Steuerung einer Therapievorrichtung bereit, die einen ersten Frequenzgenerator und einen zweiten Frequenzgenerator umfasst. Die Schritte des Verfahrens lauten Steuern des ersten Frequenzgenerators zur Erzeugung einer ersten Wechselspannung mit einer ersten Frequenz, einer ersten Spannung und einer ersten Phase, Steuern des zweiten Frequenzgenerators zur Erzeugung einer zweiten Wechselspannung mit einer zweiten Frequenz, einer zweiten Spannung und einer zweiten Phase, und Einstellen der ersten Phase im Vergleich zu der zweiten Phase, dass sich die Phasen um 160° bis 200°, insbesondere um 180°, unterscheiden.

Vorzugsweise lassen sich damit die oben beschriebene Invertierung und die damit verbundenen Erfolge erreichen. Das Verfahren ist bevorzugt dazu geeignet, die Grenzfrequenz doppelt so groß ist wie die untere Grenzfrequenz, und wobei die obere Grenzfrequenz nicht Teil des Frequenzbereichs ist.

Vorzugsweise lassen sich damit die oben beschriebene Invertierung und die damit verbundenen Erfolge erreichen. Das Verfahren ist bevorzugt dazu geeignet, die Therapievorrichtung, wie sie oben oder in Anspruch 1 definiert ist, zu steuern. Dann entspricht der erste und zweite Frequenzgenerator dem ersten und zweiten Frequenzgenerator der ersten und zweiten Frequenzgeneratoreinheit. Ferner kann das Verfahren auch dazu verwendet werden, drei Frequenzgeneratoren zu steuern. In einem solchen Fall unterscheidet sich dann die Phase der von einem dritten Frequenzgenerator (einer dritten Frequenzgeneratoreinheit) erzeugte Wechselspannung zu der ersten und zweiten Wechselspannung um 0°, 30°, 60°, 90°, 120°, 150°, 180° und/oder dazwischen liegende Werte.

Es ist bevorzugt, dass sich das Verfahren durch Einstellen der ersten Frequenz und der zweiten Frequenz, so dass das Verhältnis der ersten Frequenz zu der zweiten Frequenz gleich $2^Z$ ist, wobei z eine ganze Zahl ist, auszeichnet.

Dieses Verhältnis, so wie es bereits oben beschrieben wurde, hat sich in der Praxis durch die Erzielung besonders guter Behandlungserfolge bewährt.

Bevorzugt hat das Verfahren den Schritt Einstellen der ersten Frequenz mit einem Verhältnis von 1, 14:13, 9:8, 6:5, 5:4, 4:3, 7:5, 3:2, 8:5, 5:3, 9:5 oder 13:7 zu der unteren Grenzfrequenz. Vorzugsweise gelten für diesen Schritt auch die hinsichtlich der Therapievorrichtung angestellten Überlegengen und die genannten Merkmale.

Die Schritte eines Verfahrens zur Steuerung einer Therapievorrichtung, die einen ersten Frequenzgenerator und einen zweiten Frequenzgenerator umfasst, lauten Steuern des ersten Frequenzgenerators zur Erzeugung einer ersten Wechselspannung mit einer ersten Frequenz, einer ersten Spannung und einer ersten Phase, Steuern des zweiten Frequenzgenerators zur Erzeugung einer zweiten Wechselspannung mit einer zweiten Frequenz, einer zweiten Spannung und einer zweiten Phase, und Aktivieren des Frequenzgenerators für einen ersten Zeitraum quenzgenerators für einen ersten Zeitraum und Deaktivieren des Frequenzgenerators für einen zweiten Zeitraum, wobei vorzugsweise sich der erste Zeitraum und der zweite Zeitraum periodisch abwechseln.

Bevorzugt weist das Verfahren die oben beschriebenen Merkmale und/oder Vorteile auf. Ferner gelten vorzugsweise die hinsichtlich der Therapievorrichtung angestellten Überlegungen und Merkmale.

Die zwölf Frequenzen entsprechen in Ihrem Frequenzverhältnis bevorzugt dem Frequenzverhältnis der Töne einer Tonleiter, wie dies zuvor beschrieben wurde. Vorzugsweise hat wird die Frequenz des zweiten Frequenzgenerators als das $2^Z$-fache der Frequenz des ersten Frequenzgenerators eingestellt.

Im Folgenden wird die Erfindung anhand eines Ausführungsbeispiels in Verbindung mit den Zeichnungen beschrieben. Dabei zeigt:

Fig. 1          eine schematische Darstellung einer Therapievorrichtung;

Fig. 2          eine schematische Darstellung der Therapievorrichtung bei Behandlung eines Patienten

Fig.3           eine perspektivische Ansicht der Therapievorrichtung;

Fig. 4          eine weitere perspektivische Ansicht der Therapievorrichtung;

Fig. 5          eine Ansicht einer Anzeigeeinrichtung der Therapievorrichtung; und

Fig. 6a, b und c     eine Tabelle der in einer Steuereinrichtung der Therapievorrichtung gespeicherten Frequenzen.

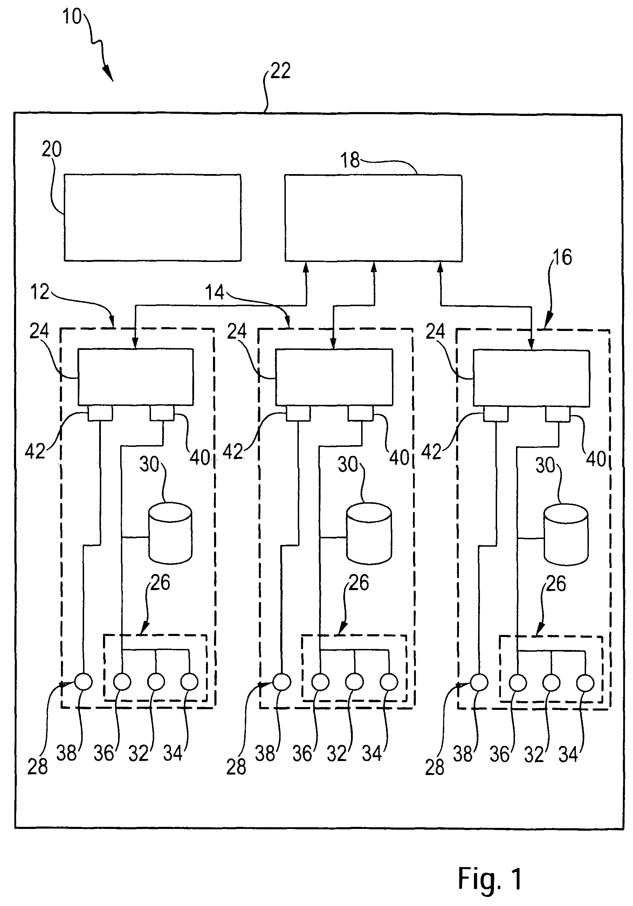

Bezug nehmend auf Fig. 1 hat eine Therapievorrichtung 10 eine erste Frequenzgeneratoreinheit 12, eine zweite Frequenzgeneratoreinheit 14, eine dritte Frequenzgeneratoreinheit 16, eine Steuereinrichtung 18 und einen Energiespeicher 20. Die genannten Elemente der Therapievorrichtung 10 sind in einem Gehäuse 22 angeordnet. Die drei Frequenzgeneratoreinheiten 12, 14 und 16 sind gegeneinander elektrisch isoliert und jeweils nur mit der Steuereinrichtung 18 verbunden.

Stellvertretend für die drei Frequenzgeneratoreinheiten soll die erste Frequenzgeneratoreinheit beschrieben werden. Die erste Frequenzgeneratoreinheit 12 hat einen Frequenzgenerator 24, einen ersten Elektrodenausgang 26, einen zweiten Elektrodenausgang 28 sowie einen Behälter 30. Der erste Elektrodenausgang 26 hat einen Handelektrodenanschluss 32, einen Abgreifelektrodenanschluss 34 und einen Leiteranschluss 36.

Der zweite Elektrodenausgang hat einen Neutralanschluss 38. An die Anschlüsse 32, 34, 36 und 38 kann jeweils eine Elektrode 39 angeschlossen werden. Der Leiter-Anschluss 36 und der Neutralanschluss 38 bilden einen BNC-Anschluss, wobei der Neutralanschluss 38 der Außenleiter des BNC-Anschlusses ist.

Der Frequenzgenerator 24 hat einen Spannungsausgang 40, an dem die von dem Frequenzgenerator 24 erzeugte Wechselspannung anliegt, und einen Neutralausgang 42, der elektrisch neutral ist. Der Spannungsausgang 40 ist mit einem Behälter 30 und dem ersten Elektrodenausgang 26 verbunden. Demnach ist der Spannungsausgang 40 mit dem Handelektrodenanschluss 32, dem Abgreifelektrodenanschluss 34 und dem BNC-Anschluss 36 verbunden. Der Neutralausgang 42 ist mit dem zweiten Elektrodenausgang 28 und damit auch mit dem Neutralanschluss 38 verbunden.

Der Behälter 30 ist aus Metall hergestellt und kann mittels eines Deckels 43 verschlossen werden. Der Behälter 30 ist in dem Gehäuse 22 angeordnet. Nach Abnahme des Deckels 43 kann eine Probe in den Behälter 30 gestellt werden. An den Handelektrodenanschluss 32 kann beispielsweise eine Elektrode 39 angeschlossen werden, die von dem Patienten in der Hand gehalten werden kann. Allerdings sind auch andere Elektroden denkbar.

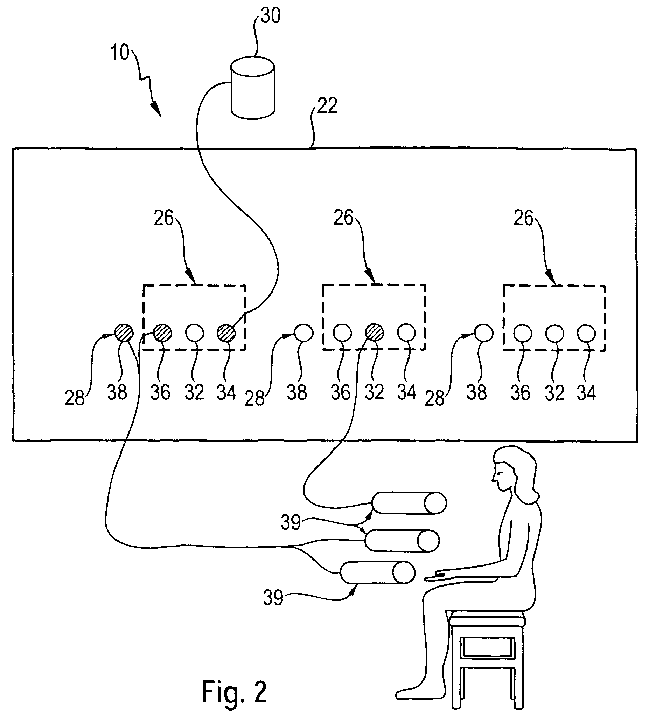

Wie in Fig. 2 zu erkennen ist, berührt ein Patient drei Elektroden 39, die als metallisches Rohr ausgeführt sind. Zwei der Elektroden 39 sind über ein BNC-Kabel mit dem BNC-Anschluss verbunden, das heißt, die beiden Elektroden sind mit dem Leiteranschluss 36 und dem Neutralanschluss verbunden. Die dritte gezeigte Elektrode 39 ist mit dem Handelektrodenanschluss 32 einer zweiten Frequenzgeneratoreinheit 12 verbunden. Die an den Elektroden anliegende Spannung kann sich in ihrer Art, Amplitude und Phase unterscheiden.

An dem Abgreifelektrodenanschluss 34 kann beispielsweise ein weiterer Behälter 30 zur Aufnahme von Proben angeschlossen werden, wie dies in Fig. 2 ersichtlich ist.

Der Energiespeicher 20, der in dieser Ausführungsform ein wiederaufladbarer Akkumulator ist, ist mit der Steuereinrichtung 18 verbunden und versorgt diese mit elektrischer Energie. Die Steuereinrichtung 18 ist wiederum mit jeder der Frequenzgeneratoreinheiten 12, 14 und 16 verbunden und steuert jeweils die erste Frequenzgeneratoreinheit 12, die zweite Frequenzgeneratoreinheit 14 und die dritte Frequenzgeneratoreinheit 16. Ferner werden die Frequenzgeneratoreinheiten 12, 14 und 16 über die Steuereinrichtung 18 mit elektrischer Energie versorgt.

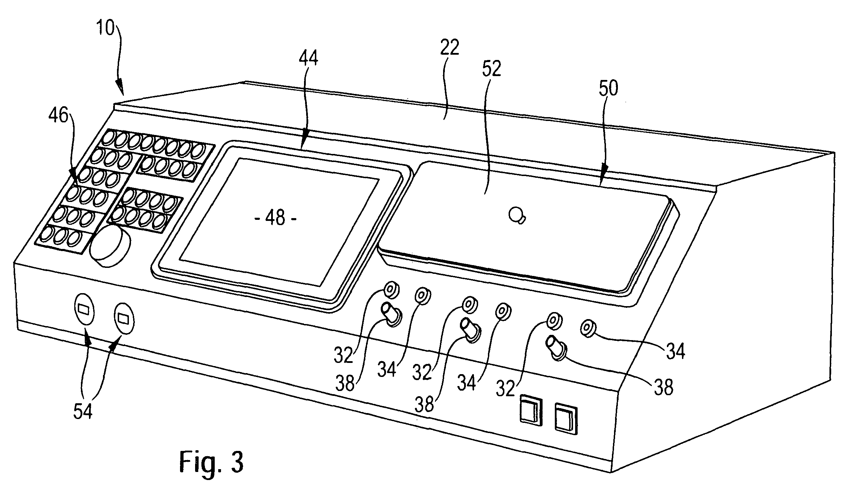

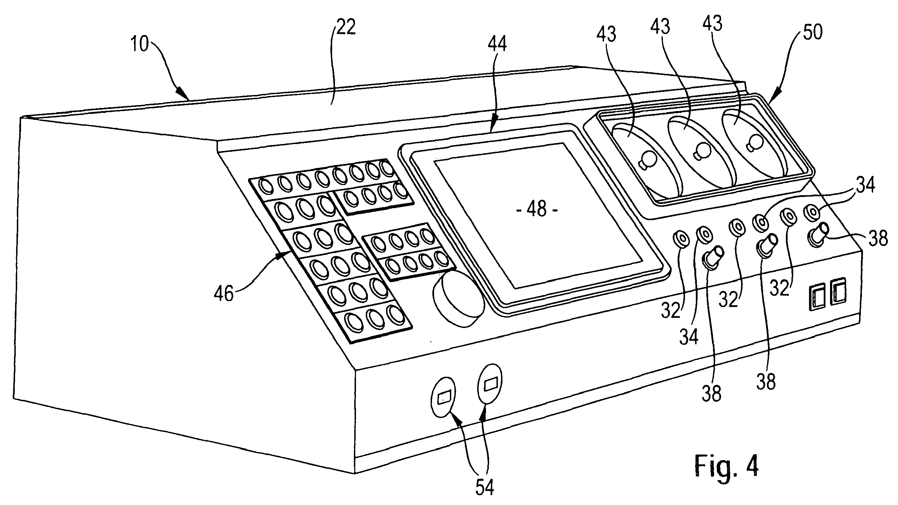

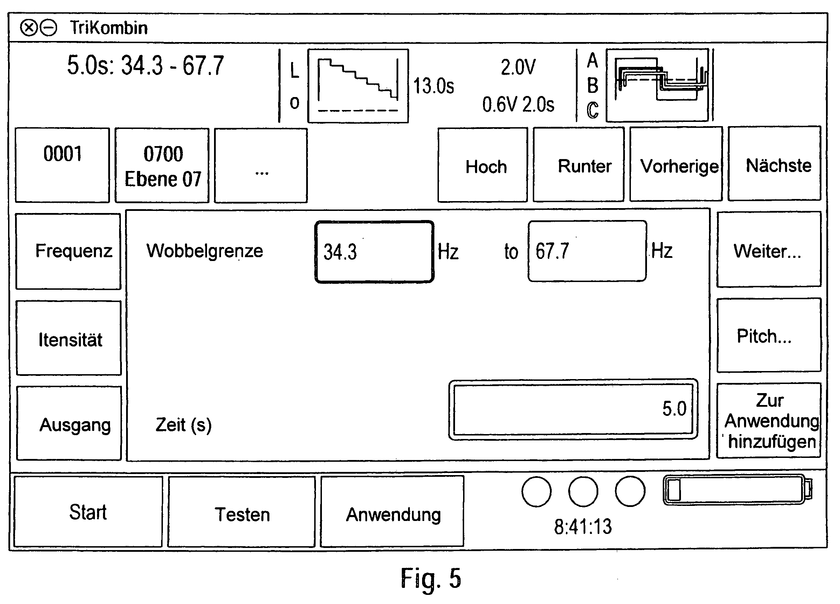

Wie aus Fig. 3 und 4 sichtbar ist, hat die Therapievorrichtung 10 ferner eine Anzeigeeinrichtung 44 und eine Eingabeeinrichtung 46. Die Anzeigeeinrichtung 44 umfasst ein Display 48, das in Fig. 5 vergrößert dargestellt ist. Auf dem Display 48 werden die Frequenz, die Spannung und die Art der Wechselspannung angegeben, die von den drei Frequenzgeneratoreinheiten 12, 14, und 16 ausgegeben wird. Ferner wird auch die Phasenbeziehung der Wechselspannung der von den drei Frequenzgeneratoreinheiten 12, 14 und 16 ausgegebenen Wechselspannungen graphisch dargestellt. Der Steuerungscomputer kann auch eine Wobbelung der Frequenz steuern, die auch auf dem Display 48 angezeigt wird. Ferner wird auch der Ladezustand des Energiespeichers 20 auf dem Display angezeigt. Die Einstellung erfolgt über Knöpfe der Eingabeeinrichtung 46. Die Tasten der Eingabeeinrichtung können sowohl eine Eingabe ermöglichen als auch durch das auf dem Display 48 angezeigte Menü führen.

Die Therapievorrichtung 10 hat auch einen Behälterzugang 50, in welchem die Behälter 30 der drei Frequenzgeneratoreinheiten 12, 14 und 16 angeordnet sind. Der Behälterzugang 50 kann durch eine Klappe 52 geschlossen werden. Die Therapievorrichtung hat außerdem einen Ladegerätanschluss 54, an welchem ein nicht gezeigtes Ladegerät angeschlossen werden kann, mittels welchem der Energiespeicher 20 geladen werden kann.

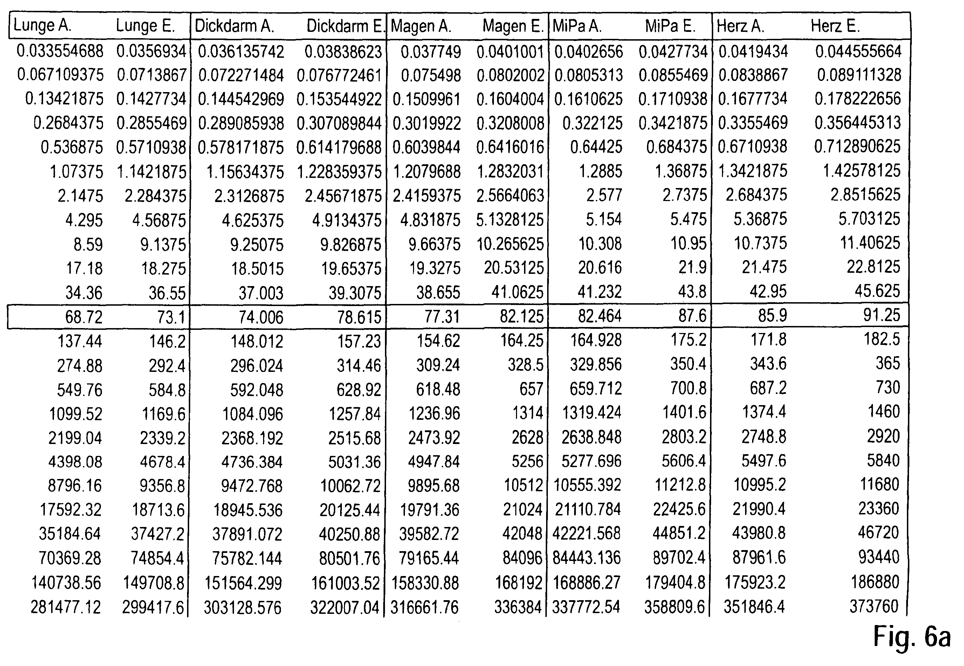

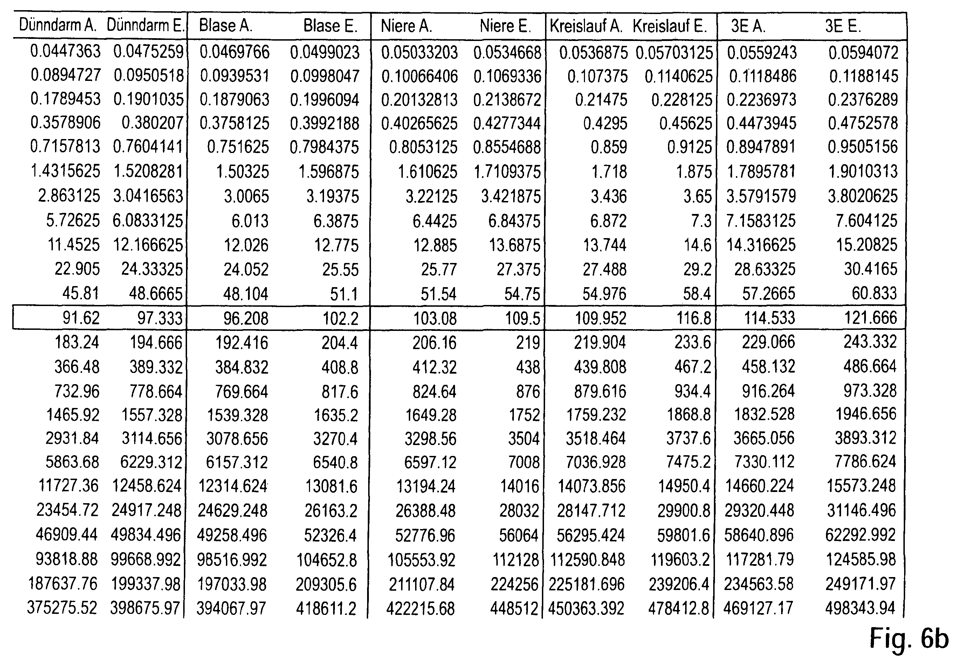

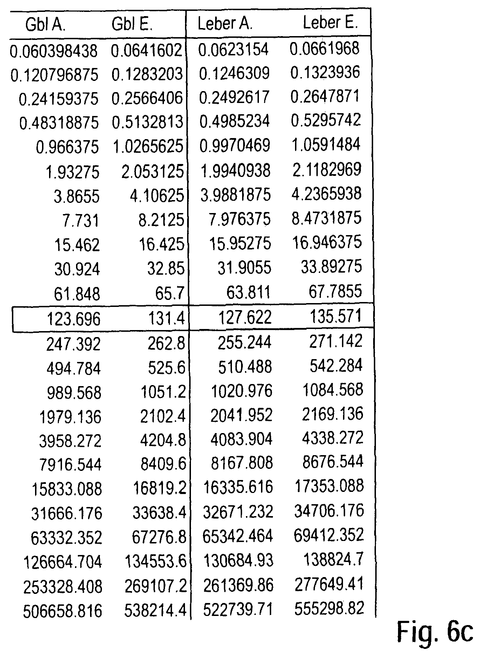

Fig. 6 zeigt eine Tabelle mit Frequenzen, die sich als besonders geeignet für die Behandlung mit elektromagnetischen Schwingungen erwiesen haben. Jede Spalte ist einem Meridian zugeordnet, der wiederum einem Organ zugeordnet ist. Jede Spalte ist ferner in zwei Unterspalten eingeteilt, wobei die linke Unterspalte die Frequenzen angibt, die mit dem Anfang des entsprechenden Meridians verknüpft sind, während die rechte Teilspalte die Frequenzen angibt, die mit dem Ende des jeweiligen Meridians verknüpft sind. Die jeweiligen Anfangs- und Endfrequenzen eines Organs sind mit den jeweiligen Anfangs- und Endfrequenzen eines anderen Organs über ein besonderes Verhältnis verknüpft. Dieses Verhältnis entspricht dem Frequenzverhältnis der Töne einer Tonleiter einer Oktave. Die Frequenzen der Lunge entsprechen dabei dem Grundton einer Tonleiter. Zur Behandlung der jeweiligen Organe wird die entsprechende Frequenz an der ersten Frequenzgeneratoreinheit ausgegeben.

Die Zeilen sind jeweils ein $2^Z$-faches der Grundfrequenz, die hervorgehoben ist. Das heißt, eine Frequenz kann durch Multiplikation mit 2, 4, 8 usw. oder mit 1/2, 1/4, 1/8 usw. erreicht werden. Bevorzugt gibt die erste Frequenzgeneratoreinheit eine Wechselspannung mit der hervorgehobenen Frequenz aus, während die zweite und dritte Frequenzgeneratoreinheit jeweils eine Wechselspannung mit einer Frequenz ausgibt, die oberhalb oder unterhalb der Frequenz der ersten Frequenzgeneratoreinheit in einer Spalte angegeben ist.

Im Folgenden soll nun die Funktionsweise der Therapievorrichtung 10 erläutert werden.

Der Verwender der Therapievorrichtung steckt die Elektroden an der Therapievorrichtung 10 an den entsprechenden Anschlüssen 32, 34, 36 und 38 ein und bringt sie in Kontakt mit der Haut des Patienten. Danach stellt der Verwender mit Hilfe der Eingabeeinrichtung 46 die Frequenz, die Spannung und die Art der von der ersten Frequenzgeneratoreinheit 12 auszugebenden Spannung ein. Die Frequenz kann durch Eintippen einer Zahl eingegeben werden oder durch Eingeben einer Nummer, die in einem Speicher der Steuereinrichtung 18 mit einer bestimmten Frequenz verknüpft ist. Der Verwender orientiert sich beispielsweise bei der zu verwendenden Frequenz an der in Fig. 6 abgebildeten Tabelle. Diese Tabelle kann auch in der Steuereinrichtung 18 gespeichert sein, so dass der Verwender lediglich die Koordinaten der zu verwendenden Frequenz einstellt, das heißt, zum einen die Nummer des Organs oder Meridians und zum anderen den gewünschten Ober- oder Unterton eingibt. Anschließend variiert er die eingestellte Frequenz, um die verwendete Frequenz an den Patienten anzupassen.

Nun stellt der Verwender ein, ob die Frequenz die von der zweiten Frequenzgeneratoreinheit 14 und der dritten Frequenzgeneratoreinheit 16 ausgegebenen Wechselspannung das Zwei-, Vier-, Acht- usw. oder Einhalb-, Einviertel oder Einachtelfache der Frequenz der von der ersten Frequenzgeneratoreinheit 12 erzeugten Wechselspannung sein soll. Der Steuerungscomputer regelt dann die von der zweiten Frequenzgeneratoreinheit 14 oder der dritten Frequenzgeneratoreinheit 16 ausgegebene Frequenz selbstständig. Auch bei einer weiteren Änderung der Frequenz der von der ersten Frequenzgeneratoreinheit 12 ausgegebenen Wechselspannung werden automatisch die Frequenzen der anderen ausgegebenen Wechselspannungen geändert.

Nun stellt der Verwender die Spannung der von den Frequenzgeneratoreinheiten 12, 14 und 16 ausgegebenen Wechselspannungen ein. Besonders geeignet ist ein Verhältnis der Spannungen von 4 : 2 : 1. Alternativ kann für den Fall, dass eine Elektrode 39 an dem Neutralanschluss 38 angeschlossen ist, auch die Stromstärke eingestellt werden, die zwischen dem ersten Elektrodenausgang 26 und dem zweiten Elektrodenausgang 28 fließen soll. Die Steuereinrichtung 18 regelt dann die Spannung der Wechselspannung so, dass die eingestellte Stromstärke fließt.

In einem nächsten Schritt wird die Art der ausgegebenen Wechselspannung für die drei Frequenzgeneratoreinheiten 12, 14 und 16 eingestellt. Der Verwender kann zwischen einer Sinus-, Rechtecks- oder Dreiecksspannung wählen.

Daraufhin wird die Phasenverschiebung der drei ausgegebenen Wechselspannungen eingestellt.

Als letzter Schritt wird die Dauer der Behandlung an der Therapievorrichtung 10 eingestellt.

## Bezugszeichenliste

| 10 | Therapievorrichtung |
| 12 | erste Frequenzgeneratoreinheit |
| 14 | zweite Frequenzgeneratoreinheit |
| 16 | dritte Frequenzgeneratoreinheit |
| 18 | Steuereinrichtung |

| | |
|---|---|
| 20 | Energiespeicher |
| 22 | Gehäuse |
| 24 | Frequenzgenerator |
| 26 | erster Elektrodenausgang |
| 28 | zweiter Elektrodenausgang |
| 30 | Behälter |
| 32 | Handelektrodenanschluss |
| 34 | Abgreifelektrodenanschluss |
| 36 | BNC-Anschluss |
| 38 | Neutralanschluss |
| 39 | Elektrode |
| 40 | Spannungsausgang |
| 42 | Neutralausgang |
| 43 | Deckel |
| 44 | Anzeigeeinrichtung |
| 46 | Eingabeeinrichtung |
| 48 | Display |
| 50 | Behälterzugang |
| 52 | Klappe |
| 54 | Ladegerätanschluss |

Aus dem Stand der Technik ist es bekannt, bioenergetische Schwingungen zur Therapie einzusetzen, um eine elektrophysikalische Behandlung eines lebenden Organismus durchzuführen. Hierzu zeigt der Stand der Technik beispielsweise in der DE 297 09 094 U1 ein Gerät für die Bioresonanz-Therapie. Bei diesem Gerät wird der Körper durch Schwingungen in Verbindung mit schwachen Strömen durchströmt, wobei eine elektrische Stimulation hervorgerufen wird. Um den Fluss von Strom durch den Körper des Patienten zu vermeiden wird in DE 20 2010 010 700 U1 eine Therapievorrichtung offenbart, bei deren Anwendung der Patient Schwingungen in eine geerdete Antenne abstrahlt. Es hat sich jedoch gezeigt, dass die aus dem Stand der Technik bekannten Vorrichtungen nicht für alle Therapiefälle wirkungsvoll einsetzbar sind.

Ein Frequenzgenerator zur Abgabe von elektromagnetischen Schwingungen an einen Patienten weist eine mit diesem bevorzugt über ein Kabel verbundene Elektrode auf, welche an einem Patienten angeordnet werden kann, beispielsweise durch Auflegen auf seine Haut.

Durch die von dem Frequenzgenerator abgegebene Frequenz wird der Organismus des zu therapierenden Patienten erregt und strahlt die als Resonanz hervorgerufene Schwingung ab.

Der Frequenzgenerator ist ausgebildet, zumindest zwölf festgelegte, unterschiedliche erste Frequenzen zu erzeugen. Diese zwölf Frequenzen liegen alle in innerhalb eines ersten Bereichs, dessen Grenzen bevorzugt einem Oktavengesetz folgen. So ist die untere Grenze gleich der niedrigsten Frequenz der zwölf ersten Frequenzen. Die obere Grenze ist durch die doppelte Frequenz der unteren Grenze gebildet, so dass sich ein oktavenspezifisches Verhältnis von 2:1 ergibt. Die obere Grenze ist aus dem ersten Bereich ausgeschlossen.

Eine derartige Therapievorrichtung stellt die Grundlage für eine effektive Behandlung eines Patienten dar. Beispielsweise können die zwölf Frequenzen den zwölf Hauptmeridianen der Traditionellen Chinesischen Medizin zugeordnet werden, wodurch es dem Behandelnden Therapeuten vereinfacht wird, diese Meridiane mit entsprechenden Frequenzen zu aktivieren.

In einer bevorzugten Ausführungsform ist der Frequenzgenerator ausgebildet, die ersten Frequenzen in konstanten Verhältnissen zueinander zu erzeugen. Die Verhältnisse müssen dabei nicht zwangsläufig identisch sein, sind jedoch bevorzugt konstant, so dass bei Variation einer der zwölf ersten Frequenzen sämtliche weiteren Frequenzen der ersten Frequenzen gemäß der bevorzugten Verhältnissen ebenso geändert werden.

Weiterhin sind die Verhältnisse der ersten Frequenzen untereinander derart ausgebildet, dass sie gleich zu den Verhältnissen sind, die die zwölf Halbtöne einer Oktave der Klanglehre aufweisen. Durch diese Gesetzmäßigkeiten ist es möglich, eine "Tonleiter" der ersten Frequenzen aufzustellen, die der Tonleiter einer Oktave mit 12 Halbtönen gleicht.

Weiterhin ist bevorzugt vorgesehen, dass die ersten Frequenzen zueinander ein Verhältnis aufweisen, das identisch mit einem Halbton und/oder einem Viertelton aus der Klanglehre ist. Die Therapievorrichtung kann daher diskrete Frequenzwerte abgeben, so dass dem Therapeuten die Auswahl der für den aktuellen Patienten passenden Frequenz erleichtert wird.

In einer weiteren vorteilhaften Ausführungsform ist der Frequenzgenerator ausgebildet, neben den zwölf ersten Frequenzen weitere Frequenzen zu erzeugen. Bevorzugt folgen die weiteren Frequenzen denselben Gesetzmäßigkeiten wie die ersten Frequenzen. Somit liegen die weiteren Frequenzen innerhalb eines weiteren Bereichs, der durch die niedrigste der weiteren Frequenzen nach unten begrenzt wird. Weiterhin ist auch für jeden weiteren Bereich die Obergrenze als doppelte Frequenz der Frequenz der Untergrenze definiert. Auf diese Weise sind die Voraussetzungen vorhanden, die weiteren Frequenzen analog zu aus der Klanglehre bekannten Oktaven um den ersten Bereich herum anzuordnen. Jede weitere Frequenz steht somit in einem festen Verhältnis zu einer der ersten Frequenzen, wobei die jeweils größere Frequenz um ein natürliches Vielfaches von 2 größer als die jeweils kleinere Frequenz ist. Dadurch ergibt sich ein aus der Klanglehre bekanntes Bild, in dem der gesamte Frequenzraum in eine Aneinanderreihung von oktavenähnlichen Bereichen aufgeteilt ist.

Eine derartige Oktavierung der möglichen Frequenzen, mit denen ein Patient behandelt werden kann, macht es für den Therapeuten deutlich einfacher, eine zu verwendende Frequenz für die Behandlung zu finden. So kann beispielsweise eine Frequenz, die einem bestimmten Meridian zugeordnet ist, als Ton angesehen werden, der jedoch auch in anderen Oktaven vorkommt. Dies bedeutet, dass beispielsweise eine doppelt oder halb so große Frequenz auf denselben Meridian wirkt. Dem Therapeuten steht somit offen, eine bestmöglichste Frequenz gemäß dem Oktavengesetz auszuwählen.

Die obere Grenze und/oder die untere Grenze des ersten Bereichs sind bevorzugt manuell anpassbar. Alternativ oder zusätzlich ist die obere Grenze und/oder die untere Grenze des ersten Bereichs bevorzugt mittels einer automatischen Steuereinheit einstellbar.

Die Anpassbarkeit der Grenzen erlaubt eine vorteilhafte Kalibrierung der Therapievorrichtung auf einen bestimmten Patienten. Da die ersten Frequenzen nicht bei allen Patienten exakt gleich sein müssen, kann hier der Therapeut korrigierend eingreifen und die ersten Frequenzen und damit den ersten Bereich entsprechend anpassen. Da die Verhältnisse der Frequenzen untereinander bevorzugt konstant sind, verschiebt sich somit lediglich die Frequenzlage als ganzes, während die grundsätzliche Oktavierung erhalten bleibt.

In besonders günstiger Weiterbildung ist vorgesehen, dass zumindest drei Frequenzgeneratoren verwendet werden. Diese können beispielsweise über jeweils separate Kabel und Elektroden mit dem Patienten verbunden sein. In vorteilhafter Weise können die drei Frequenzgeneratoren dazu eingesetzt werden, die gleichen Frequenzen zu erzeugen, um die gewünschte Therapiewirkung zu potenzieren.

Alternativ können die drei Frequenzgeneratoren verwendet werden, um unterschiedliche Frequenzen zu erzeugen. Dies ermöglicht beispielsweise die Erzeugung eines Dreiklangs mit drei Frequenzen, die jeweils die gleiche Position innerhalb unterschiedlicher Oktaven einnehmen, was bedeutet, dass diese in einem Verhältnis stehen, das ein Vielfaches von 2 ist. Auch dies kann die Wirkung der Therapie auf den Patienten verbessern.

Alternativ oder zusätzlich können die Betriebsspannungen der Frequenzgeneratoren gleich oder voneinander verschieden gewählt sein. Hier ergibt sich für den Therapeuten eine weitere vorteilhafte Einstellmöglichkeit, wie der Therapieerfolg verbessert werden kann.

In besonders günstiger Ausgestaltung ist vorgesehen, dass im Bereich des Kabels zwischen dem Frequenzgenerator und der Elektrode ein Behälter zur Aufnahme einer Substanz oder einer Probe angeordnet ist. Dieser Behälter kann beispielsweise in Form eines Bechers ausgebildet sein, in welchen chemische Substanzen, Nosoden, Organproben oder Ähnliches eingebracht sind. Diese modulieren die Frequenz des Frequenzgenerators. Bei Verwendung mehrerer Frequenzgeneratoren mit mehreren derartigen Behältern ist es beispielsweise möglich, unterschiedliche Organe des Patienten zu therapieren oder unterschiedliche Belastungen des Patienten zu berücksichtigen, beispielsweise zur Behandlung eines inneren Organs und zur Ausleitung von Giften aus dem Körper des Patienten.

Es ist besonders günstig, wenn der Frequenzgenerator Sinuswellen, Rechteckwellen oder beliebige andere Wellen erzeugt, die beispielsweise in Frequenzen von 0,01 Hz bis 40 MHz liegen. Dabei werden Spannungen von ± 0,1 V bis ± 20 V angelegt.

Es ist auch in günstiger Ausgestaltung möglich, einen Frequenzgenerator als Wobbelgenerator auszubilden. Derartige Wobbelgeneratoren oder Wobbler erzeugen eine Ausgangsfrequenz durch Mischung verschiedener Frequenzen, wobei durch Programmsteuerung eine Wobbelung realisiert werden kann, beispielsweise in Form eines Sägezahn- oder Rechteckprofils.

Diese als Resonanz auftretende Strahlung oder Erregung des Organismus kann bevorzugt von einer Antenne aufgenommen werden, welche die elektromagnetischen Schwingungen aufnimmt und erdet. Die Antenne ist in besonders vorteilhafter Weise in Form einer Teslaantenne ausgebildet, welche in besonders günstiger Weise die vom Körper des Patienten gesendeten Frequenzbereiche oder Frequenzen aufnimmt oder erdet.

Die bevorzugt vorgesehene Antenne ist bevorzugterweise in einem Abstand von 20 bis 50 cm vom zu therapierenden Patienten angeordnet. Der Patient reagiert somit auf die Erregung durch die aufgebrachten Frequenzen und sendet ein ihm eigenes Frequenzmuster ab, welches durch die Antenne aufgenommen und geerdet wird.

Im Folgenden wird die Vorrichtung anhand eines Ausführungsbeispiels in Verbindung mit der Zeichnung beschrieben. Dabei zeigt:

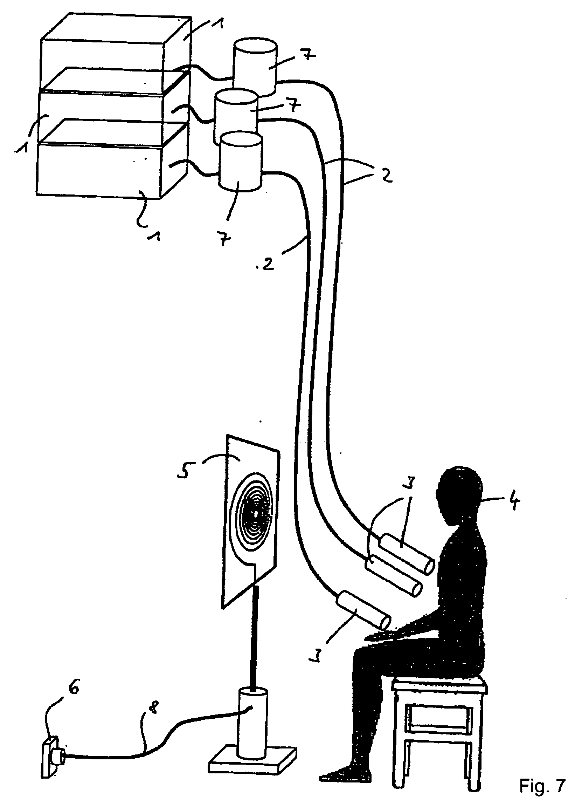

Fig.7 eine schematische Darstellung der Therapievorrichtung.

Fig.7 wird anhand der in der folgenden Bezugszeichenliste aufgeführten Bezugszeichen beschrieben.

Bezugszeichenliste

1     Frequenzgenerator

2     Kabel

3     Elektrode

4     Patient

5     Antenne

6     Erdung

7     Behälter

8     Kabel

Das Ausführungsbeispiel der Fig.7 zeigt drei Frequenzgeneratoren 1, welche über Kabel 2 mit Elektroden 3 verbunden sind. Die Kabel 2 sind als flexible elektrisch leitende Kabel ausgebildet, die Elektroden 3 sind aus Metall gefertigt oder haben zumindest metallische Kontaktflächen oder Kontaktflächen aus anderen leitenden Materialien, um an die Haut des Patienten angelegt werden zu können, so dass die von den Frequenzgeneratoren abgegebenen Frequenzen (Schwingungen) auf den Körper des Patienten übertragbar sind.

In den Kabeln 2 sind jeweils Behälter 7 zwischengeschaltet, welche beispielsweise als Becher ausgebildet sein können, um Arzneimittel, Proben, chemische Stoffe oder Ähnliches aufzunehmen.

In einer besonders vorteilhaften Anwendung des Therapiegerät aber nicht vorgesehen ist in direkter Zuordnung zu dem Patienten 4 eine Antenne 5 angeordnet, welche bevorzugterweise als Teslaantenne ausgebildet ist und über ein Kabel 8 mit einer Erdung 6 verbunden ist. Die Antenne 5 kann, wie in Fig. 7 gezeigt, als singuläre Antenne ausgebildet sein, es ist jedoch auch möglich, diese räumlich um den Patienten 4 anzuordnen. Die Teslaantenne weist jeweils zumindest eine rechtsdrehende und eine linksdrehende Spule auf.

Der Frequenzgenerator (1) kann an einen Patienten (4) beispielsweise eine Frequenz von 68,72 Hz abgeben, wodurch sich positive Effekte auf die Lunge ergeben können. Die Therapievorrichtung bestimmt daraus weitere Frequenzen, wie beispielsweise 77,31 Hz, die positive Effekte auf den Magen ergeben kann. Dabei stehen diese Frequenzen in einem Verhältnis von 9/8, was dem Verhältnis d': c' der chromatischen Tonleiter entspricht. Eine weitere Frequenz von 82,46 Hz kann sich positiv auf die Milz auswirken. Diese Frequenz steht zur Frequenz der Lunge im Verhältnis 6/5, was dem Verhältnis es': c' der chromatischen Tonleiter entspricht.

Die Therapievorrichtung ist dabei zusätzlich eingerichtet, dass ein Therapeut diese Werte verändern kann. So kann beispielsweise die Frequenz der Lunge abweichend 68,70 Hz betragen. Sobald diese manuell angepasst wird, werden sämtliche anderen Frequenzen gemäß der beispielhaft genannten Verhältnisse aktualisiert. Somit steht für jeden individuellen Organismus eine optimale Oktavierung der Frequenzen zur Verfügung.

Damit ermöglicht es die Therapievorrichtung, einen Therapeuten effizient zu unterstützen. Dieser wählt eine auf den Patienten (4) und eine auf den zu behandelnden Meridian aus der Traditionellen Chinesischen Medizin abgestimmte Frequenz, wobei ihm die Therapievorrichtung sämtliche optimalen Frequenzen für die zwölf Meridiane des menschlichen Körpers innerhalb einer Oktave zur Verfügung stellt. Da diese Frequenzen den Halbtonschritten einer aus der Musik bekannten Oktave entsprechen, können dieselben Meridiane ebenso mit einer höheren oder tieferen Frequenz behandelt werden, wenn ein Oktavenverhältnis von 2:1 eingehalten wird. Auch hier werden dem Therapeuten die passenden Frequenzen von der Therapievorrichtung zur Verfügung gestellt, so dass er wirkungsvoll in Anwendung seiner Heilkunst unterstützt wird.

## Patentansprüche

1. Therapievorrichtung, umfassend eine erste Frequenzgeneratoreinheit (12) und eine zweite Frequenzgeneratoreinheit (14) zur Erzeugung von elektromagnetischen Schwingungen, wobei die erste Frequenzgeneratoreinheit (12) und die zweite Frequenzgeneratoreinheit (14) galvanisch voneinander getrennt sind und jeweils umfassen:

   einen Frequenzgenerator (24) zur Erzeugung von Wechselspannung;
   einen ersten Elektrodenausgang (26) zum Anschluss einer ersten Elektrode (39) und
   einen zweiten Elektrodenausgang (28) zum Anschluss einer zweiten Elektrode (39);
   wobei der Frequenzgenerator (24) einen Spannungsausgang (40) aufweist, an dem die von dem Frequenzgenerator (24) erzeugte Wechselspannung anliegt,
   wobei der Frequenzgenerator (24) einen Neutralausgang (42) aufweist, der elektrisch neutral ist;
   wobei der erste Elektrodenausgang (26) mit dem Spannungsausgang (40) verbunden ist und
   wobei der zweite Elektrodenausgang (28) mit dem Neutralausgang (42) verbunden ist,
   **dadurch gekennzeichnet, dass** der Frequenzgenerator (24) zur Erzeugung von zwölf festgelegten Frequenzen ausgebildet ist, wobei die Frequenzen in einem Frequenzbereich liegen, der eine untere Grenzfrequenz und eine obere Grenzfrequenz aufweist, wobei die obere Grenzfrequenz doppelt so groß ist wie die untere Grenzfrequenz, wobei die obere Grenzfrequenz nicht Teil des Frequenzbereichs ist und wobei das Verhältnis der Frequenzen zur unteren Grenzfrequenz 1, 14:13, 9:8, 6:5, 5:4, 4:3, 7:5, 3:2, 8:5, 5:3, 9:5 oder 13:7 beträgt.

2. Therapievorrichtung nach Anspruch 1, **gekennzeichnet durch** wenigstens einen Behälter (30) zur Aufnahme einer Probe, der zwischen dem Spannungsausgang (40) und dem ersten Elektrodenausgang (26) geschaltet ist und der vorzugsweise aus einem elektrisch leitenden Material hergestellt ist.

3. Therapievorrichtung nach Anspruch 1 oder 2, **dadurch gekennzeichnet, dass** der erste Elektrodenausgang (26) wenigstens zwei Anschlüsse (32, 34, 36) umfasst, wobei vorzugsweise der zweite Elektrodenausgang (28) einen Anschluss (38) umfasst, der mit einem der Anschlüsse (32, 34, 26) des ersten Elektrodenausgangs (26) eine koaxiale Steckverbindung, insbesondere einen BNC-Anschluss, bildet, wobei weiter vorzugsweise die Therapievorrichtung (10) einen Energiespeicher (20) umfasst, insbesondere einen Akkumulator, der den Frequenzgenerator (24) mit elektrischer Energie versorgt.

4. Therapievorrichtung nach einem der Ansprüche 1 bis 3, **dadurch gekennzeichnet, dass** der Frequenzgenerator (24) zur Erzeugung von Wechselspannung mit unterschiedlichen Frequenzen, insbesondere zwischen 0,1 Hz und 100 MHz, ausgebildet ist, wobei vorzugsweise der Frequenzgenerator (24) geeignet ist, die Spannung und die Art der ausgeben Wechselspannung zu verändern.

5. Therapievorrichtung nach einem der Ansprüche 1 bis 4, **gekennzeichnet durch** eine Steuereinrichtung (18), welche mit dem Frequenzgenerator (24) der ersten Frequenzgeneratoreinheit (12) und der zweiten Frequenzgeneratoreinheit (14) verbunden ist, wobei die Frequenz, die Spannung und/oder die Art der ausgegebenen Wechselspannung mittels der Steuereinrichtung (18) steuerbar ist.

6. Therapievorrichtung nach einem der Ansprüche 1 bis 5, **gekennzeichnet durch** eine Anzeigeeinrichtung (44) und/oder eine Eingabeeinrichtung (46), wobei die Anzeigeeinrichtung (44) die Frequenz, die Spannung und/oder die Art der ausgegebenen Wechselspannung anzeigt und wobei vorzugsweise die Frequenz, die Spannung und/oder die Art der ausgegebenen Wechselspannung mittels der Eingabeeinrichtung (46) einstellbar sind.

7. Therapievorrichtung nach einem der Ansprüche 1 bis 6, **gekennzeichnet durch**
eine Steuereinrichtung (18);
wobei die Steuereinrichtung (18) eingerichtet ist, den Frequenzgenerator (24) für einen ersten Zeitraum zu aktivieren und für einen zweiten Zeitraum zu deaktivieren, wobei vorzugweise sich der erste Zeitraum und der zweite Zeitraum periodisch abwechseln.

8. Therapievorrichtung nach einem der Ansprüche 1 bis 7, **gekennzeichnet durch** eine dritte Frequenzgeneratoreinheit (16), wobei vorzugsweise die zweite Frequenzgeneratoreinheit (14) und die dritte Frequenzgeneratoreinheit (16) gegeneinander und/oder gegenüber der ersten Frequenzgeneratoreinheit (12) galvanisch getrennt sind, wobei weiter vorzugsweise die Steuereinrichtung (18) geeignet ist, die Frequenz, die Spannung und/oder die Art der ausgegebenen Wechselspannung der Frequenzgeneratoren (24) der dritten Frequenzgeneratoreinheit (16) zu steuern.

9. Therapievorrichtung nach Anspruch 8, **dadurch gekennzeichnet, dass** die Phase der von dem Frequenzgenerator (24) der zweiten Frequenzgeneratoreinheit (14) ausgegebenen Wechselspannung und/oder der von dem Frequenzgenerator (24) der dritten Frequenzgeneratoreinheit (16) ausgegebenen Wechselspannung zur Phase der von dem Frequenzgenerator (24) der ersten Frequenzgeneratoreinheit (12) ausgegebenen Wechselspannung verschoben ist, insbesondere um 180°.

10. Therapievorrichtung nach Anspruch 8 oder 9, **dadurch gekennzeichnet, dass** sich die Höhe der von dem Frequenzgenerator (24) der zweiten Frequenzgeneratoreinheit (14) ausgegebenen Wechselspannung und/oder der von dem Frequenzgenerator (24) der dritten Frequenzgeneratoreinheit (16) ausgegebenen Wechselspannung von der Höhe der von dem Frequenzgenerator (24) der ersten Frequenzgeneratoreinheit (12) ausgegebenen Wechselspannung unterscheidet.

11. Therapievorrichtung nach einem der Ansprüche 1 bis 10, **dadurch gekennzeichnet, dass** die Steuereinrichtung (18) einen Regler umfasst, welcher die Spannung der Wechselspannung derart regelt, dass der Stromfluss zwischen dem ersten Elektrodenausgang (26) und dem zweiten Elektrodenausgang (28) zumindest im Mittel konstant ist, wobei vorzugsweise die Stromstärke einstellbar ist.

**Claims**

1. A therapy device comprising a first frequency generator unit (12) and a second frequency generator unit (14) for generating electromagnetic oscillations, the first frequency generator unit (12) and the second frequency generator unit (14) being galvanically isolated from one another and comprising each:

   a frequency generator (24) for generating alternating voltage;
   a first electrode output (26) for connecting a first electrode (39) and
   a second electrode output (28) for connecting a second electrode (39);
   wherein the frequency generator (24) includes a voltage output (40) to which the alternating voltage generated by the frequency generator (24) is applied,
   wherein the frequency generator (24) includes a neutral output (42) which is electrically neutral;
   wherein the first electrode output (26) is connected with the voltage output (40) and
   wherein the second electrode output (28) is connected with the neutral output (42),
   **characterised in that** the frequency generator (24) is configured to generate twelve predetermined frequencies, wherein the frequencies are in a frequency range having a lower limit frequency and an upper limit frequency, wherein the upper limit frequency is twice as high as the lower limit frequency, wherein the upper limit frequency is not part of the frequency range and wherein the ratio of the frequencies to the lower limit frequency is 1, 14:13, 9:8, 6:5, 5:4, 4:3, 7:5, 3:2, 8:5, 5:3, 9:5 or 13:7.

2. The therapy device according to claim 1, **characterised by** at least one container (30) for receiving a sample interconnected between the voltage output (40) and the first electrode output (26) and preferably made of an electrically conductive material.

3. The therapy device according to claim 1 or 2, **characterised in that** the first electrode output (26) comprises at least two connections (32, 34, 36), wherein preferably the second electrode output (28) comprises a connection (38) that forms a coaxial plug connection, particularly a BNC connection, with one of the connections (32, 34, 36) of the first electrode output (26), wherein further preferably the therapy device (10) comprises an energy storage (20), particularly an accumulator, supplying the frequency generator (24) with electric energy.

4. The therapy device according to any of claims 1 to 3, **characterised in that** the frequency generator (24) is configured to generate alternating voltage having different frequencies, particularly between 0,1 Hz and 100 MHz, wherein preferably the frequency generator (24) is capable of regulating the voltage and the type of the output alternating voltage.

5. The therapy device according to any of claims 1 to 4, **characterised by** a control device (18) which is connected with the frequency generator (24) of the first frequency generator unit (12) and the second frequency generator unit (14), wherein the frequency, the voltage and/or the type of the output alternating voltage is controllable by means of the control device (18).

6. The therapy device according to any of claims 1 to 5, **characterised by** a display device (44) and/or an input device (46), wherein the display device (44) displays the frequency, the voltage and/or the type of the output alternating voltage and wherein preferably the frequency, the voltage and/or the type of the output alternating voltage are adjustable by means of the input device (46).

7. The therapy device according to any of claims 1 to 6, **characterised by**
   a control device (18),
   wherein the control device (18) is adapted to activate the frequency generator (24) for a first period and to deactivate the frequency generator (24) for a second period, wherein preferably the first period alternates periodically with the second period.

8. The therapy device according to any of claims 1 to 7, **characterised by** a third frequency generator unit (16), wherein preferably the second frequency generator unit (14) and the third frequency generator unit (16) are galvanically isolated from one another and/or from the first frequency generator unit (12), wherein further preferably the control device (18) is capable of controlling the frequency, the voltage and/or the type of the output alternating voltage of the frequency generators (24) of the third frequency generator unit (16).

9. The therapy device according to claim 8, **characterised in that** the phase of the alternating voltage output from the frequency generator (24) of the second frequency generator unit (14) and/or the alternating voltage output from the frequency generator (24) of the third frequency generator unit (16) is shifted from the phase of the alternating voltage output from the frequency generator (24) of the first frequency generator unit (12), particularly by 180°.

10. The therapy device according to claim 8 or 9, **characterised in that** the level of the alternating voltage output from the frequency generator (24) of the second frequency generator unit (14) and/or the alternating voltage output from the frequency generator (24) of the third frequency generator unit (16) differs from the level of the alternating voltage output from the frequency generator (24) of the first frequency generator unit (12).

11. The therapy device according to any of claims 1 to 10, **characterised in that** the control device (18) comprises a controller which controls the voltage of the alternating voltage in such a way that the current flow between the first electrode output (26) and the second electrode output (28) is constant, at least on average, wherein preferably the amperage is adjustable.

**Revendications**

1. Appareil de thérapie incluant un premier ensemble générateur de fréquence (12) et un second ensemble générateur de fréquence (14) pour générer des vibrations électromagnétiques, dans lequel le premier ensemble générateur de fréquence (12) et le second ensemble générateur de fréquence (14) sont séparés l'un de l'autre par voie galvanique et incluent chacun :

   un générateur de fréquence (24) pour générer une tension alternative ;
   une première sortie d'électrode (26) pour connecter une première électrode (39) et
   une seconde sortie d'électrode (28) pour connecter une seconde électrode (39) ;
   le générateur de fréquence (24) présentant une sortie de tension (40) à laquelle s'applique la tension alternative générée par le générateur de fréquence (24),

le générateur de fréquence (24) présentant une sortie neutre (42) qui est électriquement neutre ;
la première sortie d'électrode (26) étant reliée à la sortie de tension (40) et
la seconde sortie d'électrode (28) étant reliée à la sortie neutre (42),
**caractérisé en ce que** le générateur de fréquence (24) est conçu pour générer douze fréquences déterminées, les fréquences se situant dans une plage de fréquences qui présente une fréquence limite inférieure et une fréquence limite supérieure, la fréquence limite supérieure étant deux fois plus élevée que la fréquence limite inférieure, la fréquence limite supérieure ne faisant pas partie de la plage de fréquences et la relation des fréquences par rapport à la fréquence limite inférieure étant de 1, de 14:13, de 9:8, de 6:5, de 5:4, de 4:3, de 7:5, de 3:2, de 8:5, de 5:3, de 9:5 ou de 13:7.

2. Appareil de thérapie selon la revendication 1, **caractérisé par** au moins un récipient (30) pour recevoir un échantillon, qui est monté entre la sortie de tension (40) et la première sortie d'électrode (26) et est réalisé de préférence en un matériau électriquement conducteur.

3. Appareil de thérapie selon la revendication 1 ou 2, **caractérisé en ce que** la première sortie d'électrode (26) inclut au moins deux bornes (32, 34, 36), et de préférence la seconde sortie d'électrode (28) inclut une borne (38) qui forme une connexion enfichable coaxiale, en particulier une connexion BNC, avec l'une des bornes (32, 34, 26) de la première sortie d'électrode (26), et l'appareil de thérapie (10) inclut de préférence un réservoir d'énergie (20), en particulier un accumulateur qui alimente en énergie électrique le générateur de fréquence (24).

4. Appareil de thérapie selon l'une des revendications 1 à 3, **caractérisé en ce que** le générateur de fréquence (24) est conçu de préférence pour générer une tension alternative à des fréquences différentes, comprises en particulier entre 0,1 Hz et 100 MHz, et de préférence le générateur de fréquence (24) est apte à modifier la tension et le type de tension alternative délivrée.

5. Appareil de thérapie selon l'une des revendications 1 à 4, **caractérisé par** un moyen de commande (18) qui est connecté au générateur de fréquence (24) du premier ensemble générateur de fréquence (12) et du second ensemble générateur de fréquence (14), la fréquence, la tension et/ou le type de tension alternative délivrée étant contrôlables par le moyen de commande (18).

6. Appareil de thérapie selon l'une des revendications 1 à 5, **caractérisé par** un moyen d'affichage (44) et/ou par un moyen de saisie (46), le moyen d'affichage (44) affichant la fréquence, la tension et/ou le type de tension alternative délivrée, et de préférence la fréquence, la tension et/ou le type de tension alternative délivrée sont réglables par le moyen de saisie (46).

7. Appareil de thérapie selon l'une des revendications 1 à 6, **caractérisé par**
un moyen de commande (18) ;
le moyen de commande (18) étant conçu pour activer le générateur de fréquence (24) pendant un premier intervalle temporel et pour le désactiver pendant un second intervalle temporel, et de préférence le premier intervalle temporel et le second intervalle temporel alternent périodiquement.

8. Appareil de thérapie selon l'une des revendications 1 à 7, **caractérisé par** un troisième ensemble générateur de fréquence (16), et de préférence le second ensemble générateur de fréquence (14) et le troisième ensemble générateur de fréquence (16) sont séparés par voie galvanique l'un par rapport à l'autre et/ou par rapport au premier ensemble générateur de fréquence (12), et de préférence le moyen de commande (18) est en outre apte à commander la fréquence, la tension et/ou le type de tension alternative délivrée des générateurs de fréquence (24) du troisième ensemble générateur de fréquence (16).

9. Appareil de thérapie selon la revendication 8, **caractérisé en ce que** la phase de la tension alternative délivrée par le générateur de fréquence (24) du second ensemble générateur de fréquence (14) et/ou celle de la tension alternative délivrée par le générateur de fréquence (24) du troisième ensemble générateur de fréquence (16) est décalée, en particulier de 180°, par rapport à la phase de la tension alternative délivrée par le générateur de fréquence (24) du premier ensemble générateur de fréquence (12).

10. Appareil de thérapie selon la revendication 8 ou 9, **caractérisé en ce que** la hauteur de la tension alternative délivrée par le générateur de fréquence (24) du second ensemble générateur de fréquence (14) et/ou celle de la tension alternative délivrée par le générateur de fréquence (24) du troisième ensemble générateur de fréquence (16) se distingue de la hauteur de la tension alternative délivrée par le générateur de fréquence (24) du premier ensemble générateur de fréquence (12).

**11.** Appareil de thérapie selon l'une des revendications 1 à 10, **caractérisé en ce que** le moyen de commande (18) inclut un régulateur qui régule la valeur de la tension alternative de telle sorte que le courant électrique entre la première sortie d'électrode (26) et la seconde sortie d'électrode (28) est constant au moins en moyenne, l'intensité du courant étant de préférence réglable.

Fig. 1

Fig. 2

Fig. 3

EP 2 709 718 B1

Fig. 4

⊗⊖ TriKombin

5.0s: 34.3 - 67.7   L   13.0s   2.0V   A
                    0              0.6V 2.0s   B
                                              C

| 0001 | 0700 Ebene 07 | ... | | Hoch | Runter | Vorherige | Nächste |

| Frequenz | Wobbelgrenze | 34.3 | Hz | to | 67.7 | Hz | Weiter... |

Itensität

Pitch...

| Ausgang | Zeit (s) | | 5.0 | Zur Anwendung hinzufügen |

| Start | Testen | Anwendung | 8:41:13 |

Fig. 5

| Lunge A. | Lunge E. | Dickdarm A. | Dickdarm E. | Magen A. | Magen E. | MiPa A. | MiPa E. | Herz A. | Herz E. |
|---|---|---|---|---|---|---|---|---|---|
| 0.033554688 | 0.0356934 | 0.036135742 | 0.03838623 | 0.037749 | 0.0401001 | 0.0402656 | 0.0427734 | 0.0419434 | 0.044555664 |
| 0.067109375 | 0.0713867 | 0.072271484 | 0.076772461 | 0.075498 | 0.0802002 | 0.0805313 | 0.0855469 | 0.0838867 | 0.089111328 |
| 0.13421875 | 0.1427734 | 0.144542969 | 0.153544922 | 0.1509961 | 0.1604004 | 0.1610625 | 0.1710938 | 0.1677734 | 0.178222656 |
| 0.2684375 | 0.2855469 | 0.289085938 | 0.307089844 | 0.3019922 | 0.3208008 | 0.322125 | 0.3421875 | 0.3355469 | 0.356445313 |
| 0.536875 | 0.5710938 | 0.578171875 | 0.614179688 | 0.6039844 | 0.6416016 | 0.64425 | 0.684375 | 0.6710938 | 0.712890625 |
| 1.07375 | 1.1421875 | 1.15634375 | 1.228359375 | 1.2079688 | 1.2832031 | 1.2885 | 1.36875 | 1.3421875 | 1.42578125 |
| 2.1475 | 2.284375 | 2.3126875 | 2.45671875 | 2.4159375 | 2.5664063 | 2.577 | 2.7375 | 2.684375 | 2.8515625 |
| 4.295 | 4.56875 | 4.625375 | 4.9134375 | 4.831875 | 5.1328125 | 5.154 | 5.475 | 5.36875 | 5.703125 |
| 8.59 | 9.1375 | 9.25075 | 9.826875 | 9.66375 | 10.265625 | 10.308 | 10.95 | 10.7375 | 11.40625 |
| 17.18 | 18.275 | 18.5015 | 19.65375 | 19.3275 | 20.53125 | 20.616 | 21.9 | 21.475 | 22.8125 |
| 34.36 | 36.55 | 37.003 | 39.3075 | 38.655 | 41.0625 | 41.232 | 43.8 | 42.95 | 45.625 |
| 68.72 | 73.1 | 74.006 | 78.615 | 77.31 | 82.125 | 82.464 | 87.6 | 85.9 | 91.25 |
| 137.44 | 146.2 | 148.012 | 157.23 | 154.62 | 164.25 | 164.928 | 175.2 | 171.8 | 182.5 |
| 274.88 | 292.4 | 296.024 | 314.46 | 309.24 | 328.5 | 329.856 | 350.4 | 343.6 | 365 |
| 549.76 | 584.8 | 592.048 | 628.92 | 618.48 | 657 | 659.712 | 700.8 | 687.2 | 730 |
| 1099.52 | 1169.6 | 1084.096 | 1257.84 | 1236.96 | 1314 | 1319.424 | 1401.6 | 1374.4 | 1460 |
| 2199.04 | 2339.2 | 2368.192 | 2515.68 | 2473.92 | 2628 | 2638.848 | 2803.2 | 2748.8 | 2920 |
| 4398.08 | 4678.4 | 4736.384 | 5031.36 | 4947.84 | 5256 | 5277.696 | 5606.4 | 5497.6 | 5840 |
| 8796.16 | 9356.8 | 9472.768 | 10062.72 | 9895.68 | 10512 | 10555.392 | 11212.8 | 10995.2 | 11680 |
| 17592.32 | 18713.6 | 18945.536 | 20125.44 | 19791.36 | 21024 | 21110.784 | 22425.6 | 21990.4 | 23360 |
| 35184.64 | 37427.2 | 37891.072 | 40250.88 | 39582.72 | 42048 | 42221.568 | 44851.2 | 43980.8 | 46720 |
| 70369.28 | 74854.4 | 75782.144 | 80501.76 | 79165.44 | 84096 | 84443.136 | 89702.4 | 87961.6 | 93440 |
| 140738.56 | 149708.8 | 151564.299 | 161003.52 | 158330.88 | 168192 | 168886.27 | 179404.8 | 175923.2 | 186880 |
| 281477.12 | 299417.6 | 303128.576 | 322007.04 | 316661.76 | 336384 | 337772.54 | 358809.6 | 351846.4 | 373760 |

Fig. 6a

EP 2 709 718 B1

| Dünndarm A. | Dünndarm E. | Blase A. | Blase E. | Niere A. | Niere E. | Kreislauf A. | Kreislauf E. | 3E A. | 3E E. |
|---|---|---|---|---|---|---|---|---|---|
| 0.0447363 | 0.0475259 | 0.0469766 | 0.0499023 | 0.05033203 | 0.0534668 | 0.0536875 | 0.05703125 | 0.0559243 | 0.0594072 |
| 0.0894727 | 0.0950518 | 0.0939531 | 0.0998047 | 0.10066406 | 0.1069336 | 0.107375 | 0.1140625 | 0.1118486 | 0.1188145 |
| 0.1789453 | 0.1901035 | 0.1879063 | 0.1996094 | 0.20132813 | 0.2138672 | 0.21475 | 0.228125 | 0.2236973 | 0.2376289 |
| 0.3578906 | 0.380207 | 0.3758125 | 0.3992188 | 0.40265625 | 0.4277344 | 0.4295 | 0.45625 | 0.4473945 | 0.4752578 |
| 0.7157813 | 0.7604141 | 0.751625 | 0.7984375 | 0.8053125 | 0.8554688 | 0.859 | 0.9125 | 0.8947891 | 0.9505156 |
| 1.4315625 | 1.5208281 | 1.50325 | 1.596875 | 1.610625 | 1.7109375 | 1.718 | 1.875 | 1.7895781 | 1.9010313 |
| 2.863125 | 3.0416563 | 3.0065 | 3.19375 | 3.22125 | 3.421875 | 3.436 | 3.65 | 3.5791579 | 3.8020625 |
| 5.72625 | 6.0833125 | 6.013 | 6.3875 | 6.4425 | 6.84375 | 6.872 | 7.3 | 7.1583125 | 7.604125 |
| 11.4525 | 12.166625 | 12.026 | 12.775 | 12.885 | 13.6875 | 13.744 | 14.6 | 14.316625 | 15.20825 |
| 22.905 | 24.33325 | 24.052 | 25.55 | 25.77 | 27.375 | 27.488 | 29.2 | 28.63325 | 30.4165 |
| 45.81 | 48.6665 | 48.104 | 51.1 | 51.54 | 54.75 | 54.976 | 58.4 | 57.2665 | 60.833 |
| 91.62 | 97.333 | 96.208 | 102.2 | 103.08 | 109.5 | 109.952 | 116.8 | 114.533 | 121.666 |
| 183.24 | 194.666 | 192.416 | 204.4 | 206.16 | 219 | 219.904 | 233.6 | 229.066 | 243.332 |
| 366.48 | 389.332 | 384.832 | 408.8 | 412.32 | 438 | 439.808 | 467.2 | 458.132 | 486.664 |
| 732.96 | 778.664 | 769.664 | 817.6 | 824.64 | 876 | 879.616 | 934.4 | 916.264 | 973.328 |
| 1465.92 | 1557.328 | 1539.328 | 1635.2 | 1649.28 | 1752 | 1759.232 | 1868.8 | 1832.528 | 1946.656 |
| 2931.84 | 3114.656 | 3078.656 | 3270.4 | 3298.56 | 3504 | 3518.464 | 3737.6 | 3665.056 | 3893.312 |
| 5863.68 | 6229.312 | 6157.312 | 6540.8 | 6597.12 | 7008 | 7036.928 | 7475.2 | 7330.112 | 7786.624 |
| 11727.36 | 12458.624 | 12314.624 | 13081.6 | 13194.24 | 14016 | 14073.856 | 14950.4 | 14660.224 | 15573.248 |
| 23454.72 | 24917.248 | 24629.248 | 26163.2 | 26388.48 | 28032 | 28147.712 | 29900.8 | 29320.448 | 31146.496 |
| 46909.44 | 49834.496 | 49258.496 | 52326.4 | 52776.96 | 56064 | 56295.424 | 59801.6 | 58640.896 | 62292.992 |
| 93818.88 | 99668.992 | 98516.992 | 104652.8 | 105553.92 | 112128 | 112590.848 | 119603.2 | 117281.79 | 124585.98 |
| 187637.76 | 199337.98 | 197033.98 | 209305.6 | 211107.84 | 224256 | 225181.696 | 239206.4 | 234563.58 | 249171.97 |
| 375275.52 | 398675.97 | 394067.97 | 418611.2 | 422215.68 | 448512 | 450363.392 | 478412.8 | 469127.17 | 498343.94 |

Fig. 6b

EP 2 709 718 B1

| Gbl A. | Gbl E. | Leber A. | Leber E. |
|---|---|---|---|
| 0.060398438 | 0.0641602 | 0.0623154 | 0.0661968 |
| 0.120796875 | 0.1283203 | 0.1246309 | 0.1323936 |
| 0.24159375 | 0.2566406 | 0.2492617 | 0.2647871 |
| 0.48318875 | 0.5132813 | 0.4985234 | 0.5295742 |
| 0.966375 | 1.0265625 | 0.9970469 | 1.0591484 |
| 1.93275 | 2.053125 | 1.9940938 | 2.1182969 |
| 3.8655 | 4.10625 | 3.9881875 | 4.2365938 |
| 7.731 | 8.2125 | 7.976375 | 8.4731875 |
| 15.462 | 16.425 | 15.95275 | 16.946375 |
| 30.924 | 32.85 | 31.9055 | 33.89275 |
| 61.848 | 65.7 | 63.811 | 67.7855 |
| 123.696 | 131.4 | 127.622 | 135.571 |
| 247.392 | 262.8 | 255.244 | 271.142 |
| 494.784 | 525.6 | 510.488 | 542.284 |
| 989.568 | 1051.2 | 1020.976 | 1084.568 |
| 1979.136 | 2102.4 | 2041.952 | 2169.136 |
| 3958.272 | 4204.8 | 4083.904 | 4338.272 |
| 7916.544 | 8409.6 | 8167.808 | 8676.544 |
| 15833.088 | 16819.2 | 16335.616 | 17353.088 |
| 31666.176 | 33638.4 | 32671.232 | 34706.176 |
| 63332.352 | 67276.8 | 65342.464 | 69412.352 |
| 126664.704 | 134553.6 | 130684.93 | 138824.7 |
| 253328.408 | 269107.2 | 261369.86 | 277649.41 |
| 506658.816 | 538214.4 | 522739.71 | 555298.82 |

Fig. 6c

EP 2 709 718 B1

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202010010700 U1
- US 2005090867 A1
- US 6424864 B1
- US 2006190063 A1
- GB 1577834 A
- US 2003176901 A1
- US 5891185 A
- EP 0293068 A1
- DE 29709094 U1